(19) Europäisches Patentamt
European Patent Office
Office européen des brevets

(11) EP 1 773 295 B1

(12) FASCICULE DE BREVET EUROPEEN

(45) Date de publication et mention de la délivrance du brevet:
**29.07.2009 Bulletin 2009/31**

(21) Numéro de dépôt: **05777196.6**

(22) Date de dépôt: **15.06.2005**

(51) Int Cl.:
***A61K 8/898*** *(2006.01)* ***A61Q 5/00*** *(2006.01)*
***A61Q 19/00*** *(2006.01)*

(86) Numéro de dépôt international:
**PCT/FR2005/001489**

(87) Numéro de publication internationale:
**WO 2006/008367 (26.01.2006 Gazette 2006/04)**

# (54) COMPOSITION COSMETIQUE COMPRENANT UN POLYORGANOSILOXANE ET SES UTILISATIONS

KOSMETISCHE ZUSAMMENSETZUNG MIT POLYORGANOSILOXAN UND IHRE ANWENDUNG

COSMETIC COMPOSITION COMPRISING POLYORGANOSILOXANE AND USES THEREOF

(84) Etats contractants désignés:
**AT BE BG CH CY CZ DE DK EE ES FI FR GB GR HU IE IS IT LI LT LU MC NL PL PT RO SE SI SK TR**

(30) Priorité: **23.06.2004 FR 0406816**

(43) Date de publication de la demande:
**18.04.2007 Bulletin 2007/16**

(73) Titulaire: **Rhodia Chimie SA**
**92512 Boulogne-Billancourt Cedex (FR)**

(72) Inventeurs:
- **MANUSZAK, Melissa**
**F-75005 PARIS (FR)**
- **LORENTZ, Gilles**
**F-69006 LYON (FR)**

(74) Mandataire: **Boittiaux, Vincent**
**Rhodia Services Dir. Pro. Ind.**
**40 Rue de la Haie Coq.**
**93300 Aubervilliers (FR)**

(56) Documents cités:
**EP-A- 1 358 864**
**WO-A-03/066007**
**WO-A-03/088939**
**FR-A- 2 840 615**
**US-A- 5 721 297**
**US-B1- 6 605 577**
**US-B2- 6 642 194**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention a pour objet une composition cosmétique comprenant un polyorganosiloxane et un vecteur cosmétiquement acceptable.

De nombreuses compositions cosmétiques comprennent des polyorganosiloxanes («silicones»). Les polyorganosiloxanes peuvent être utilisés pour procurer un effet de conditionnement des cheveux ou de la peau. Les polyorganosiloxanes peuvent également être utilisés pour des effets sensoriels, dits effets cosmétiques, lors de leur application sur la peau, les cheveux ou les lèvres.

Ainsi, il a été proposé d'utiliser dans des compostions cométiques des polyorganosiloxanes de nombreuses structures chimiques différentes, pouvant éventuellement porter différents groupes fonctionnels.

Les polydimethylorganosiloxanes (PDMS) linéaires peuvent être utilisés comme agents sensoriels sur la peau, comme agents protecteurs faisant barrière à l'eau, comme agents démoussants, comme agents éliminateurs de blancheurs crémeuses de compositions cosmétiques apparaissant lors d'un premier frottement sur la peau ou les cheveux «desoapers», comme agents conditionneurs ou comme agents émollients.

Les polyorganosiloxanes comprenant des fonctions polyether résultent d'un remplacement de groupes méthyles sur le squelettes siloxy, par des substituants polyether (polyalcoxylés), en général des substituants oxyde d'éthylène et oxyde de propylène. Cette substitution provoque une modification de la balance hydrophile/hydrophobe (HLB) du polyorganosiloxane. L'addition de groupes polyether permet une modification de la compatibilité du polyorganosiloxane avec des solvants polaires ou non polaires. Ainsi ces polyorganosiloxanes comprenant des fonctions polyether peuvent être de solubles dans l'eau à solubles dans des solvants oléophiles. Ils peuvent trouver des utilisations dans des phases aqueuses et dans des phases huileuses de compositions cosmétiques. Ils peuvent être utilisés comme émulsifiants d'émulsions eau-dans-huile, ou eau-dans-cyclopentasiloxane. Ils peuvent renforcer des effets sensoriels dans des compositions de gels-douche. Ils peuvent jouer un rôle de modificateurs de résines de sprays pour les cheveux. Ils peuvent être utilisés comme conditionneurs doux dans des compositions transparentes.

On a en outre décrit des compositions cosmétiques comprenant des polyorganosiloxanes aminés, souvent en liaison avec des bénéfices en matière de coloration. La présence des groupes aminés améliore l'affinité du polyorganosiloxane pour les cheveux et procure un bon conditionnement des cheveux, pour des compositions destinées à être rincées ou à ne pas être rincées.

Les documents US 6,605,577 (Chemsil Silicones Inc.), US 6,642,194 (Chemsil Silicones Inc.), WO 03/088939 (The Procter & Gamble Company), WO 03/066007 (Dow Coming), décrivent des compositions cosmétiques comprenant un polyorganosiloxane portant des groupes fonctionnels particuliers.

Il existe un besoin pour de nouvelles compositions, présentant, pour les consommateurs, des fonctionnalités ajoutées, améliorées ou simplifiées, et/ou, pour les producteurs, des avantages en matière de simplicité de préparation, de versatilité de formulation, de compatibilité entre ingrédients, et/ou de coûts.

La présente invention satisfait ce besoin en proposant de nouvelles compositions cosmétiques.

Ainsi, l'invention a pour objet une composition cosmétique comprenant un vecteur cosmétiquement acceptable et un polyorganosiloxane, caractérisée en ce que le polyorganosiloxane présente la formule générale (I) suivante:

$$[R_aX_bY_cSiO_{(4-a-b-c)/2}]_N \qquad (I)$$

dans laquelle:

- $R_aX_bY_cSiO_{(4-a-b-c)/2}$, identiques ou différents, représentent des motifs, linéaires ou branchés, compris dans le polyorganosiloxane,
- N représente le nombre d'atomes de silicium compris dans le polyorganosiloxane, supérieur ou égal à 3,
- a, b, et c, identiques ou différents, sont des nombres égaux à 0,1, 2 ou 3,
- a+b+c, identique ou différent, est égal à 0, 1, 2 ou 3,
- R, identique ou différent, représente un groupe alkyle en $C_1$-$C_{18}$, un groupe aryle en $C_6$-$C_{12}$, un groupe aralkyle $C_6$-$C_{12}$, un groupe alkylaryl en $C_6$-$C_{12}$, un groupe de formule
- $[CH_2]_3$-$NH[CH_2]_2$-$NH_2$, un groupe de formule -$[CH_2]_3$-$NH_2$, un groupe hydroxyle, un groupe alcoxy en $C_1$-$C_{18}$, un groupe hydroxyalkyl $C_1$-$C_{18}$, ou un groupe hydroxyalkyletheralkyl en $C_1$-$C_{18}$, lesdits groupes étant éventuellement substitués,
- X, identique ou différent, représente un groupe polyalcoxylé porté par un atome de silicium
- Y, identique ou différent, représente un groupe porté par un atome de silicium, de formule -$R^4$-U-Hals, dans laquelle:

    - $R^4$ est un groupe de liaison divalent hydrocarboné, de préférence un groupe alkyle,
    - U est une liaison covalente ou un groupe rotule divalent comprenant un hétéroatome,
    - Hals est un groupe fonctionnel comprenant une amine stériquement encombrée,

- au moins un atome de silicium du polyorganosiloxane porte un groupe X, et
- au moins un atome de silicium du polyorganosiloxane porte un groupe Y.

Le polyorganosiloxane de formule (I) peut présenter notamment une formulabilité améliorée par rapport à d'autres polyorganosiloxanes. Il peut en outre moduler, améliorer ou procurer des propriétés intéressantes en terme de:

- conditionnement,
- effets sensoriels,
- prévention du vieillissement de la peau et des cheveux,
- aspect des compositions, notamment faible jaunissement, et/ou
- autres propriétés mentionnées plus bas dans la présente description.

La composition peut comprendre en plus du polyorganosiloxane de formule (I), des ingrédients choisis parmi les suivants:

- au moins un tensioactif anionique et/ou amphotère, seul ou en mélange,
- éventuellement, au moins un agent de stabilisation et/ou de conditionnement et/ou d'aide au conditionnement, ou un mélange de tels agents,
- éventuellement un autre polyorganosiloxane,
- éventuellement un filtre UV.

Dans une première partie, on détaille certains des ingrédients pouvant être compris dans la composition cométique.

Polyorganosiloxane de formule (I)

Le polyorganosiloxane présente la formule générale (I) suivante:

$$[R_aX_bY_cSiO_{(4-a-b-c)/2}]_N \qquad (I)$$

dans laquelle:

- $R_aX_bY_cSiO_{(4-a-b-c)/2,}$ identiques ou différents, représentent des motifs, linéaires ou branchés, compris dans le polyorganosiloxane,
- N représente le nombre d'atomes de silicium compris dans le polyorganosiloxane, supérieur ou égal à 3,
- a, b, et c, identiques ou différents, sont des nombres égaux à 0, 1, 2 ou 3,
- a+b+c, identique ou différent, est égal à 0, 1, 2 ou 3,
- R, identique ou différent, représente un groupe alkyle en $C_1$-$C_{18}$, un groupe aryle en $C_6$-$C_{12}$, un groupe aralkyle $C_6$-$C_{12}$, un groupe alkylaryl en $C_6$-$C_{12}$, un groupe de formule $-[CH_2]_3-NH[CH_2]_2-NH_2$, un groupe de formule $-[CH_2]_3-NH_2$, un groupe hydroxyle, un groupe alcoxy en $C_1$-$C_{18}$, un groupe hydroxyalkyl $C_1$-$C_{18}$, ou un groupe hydroxyalkyletheralkyl en $C_1$-$C_{18}$, lesdits groupes étant éventuellement substitués,
- X, identique ou différent, représente un groupe polyalcoxylé porté par un atome de silicium,
- Y, identique ou différent, représente un groupe porté par un atome par un atome de silicium, de formule $-R^4$-U-Hals, dans laquelle:
  - $R^4$ est un groupe de liaison divalent hydrocarboné, de préférence un groupe alkyle,
  - U est une liaison covalente ou un groupe rotule divalent comprenant un hétéroatome,
  - Hals est un groupe fonctionnel comprenant une amine stériquement encombrée,
- au moins un atome de silicium du polyorganosiloxane porte un groupe X, et
- au moins un atome de silicium du polyorganosiloxane porte un groupe Y.

Dans la formule (I), les groupes X et Y sont de préférence portés par des atomes de silicium différents.
Le groupe X présente avantageusement la formule $L^X$-$Z^x$-Palc, dans laquelle:

- $L^X$ est un groupe de liaison divalent, de préférence un groupe alkyle,
- $Z^X$ est une liaison covalente ou un groupe rotule divalent comprenant un hétéroatome,
- Palc est un groupe de formule $[OE]_s$-$[OP]_t$-X', dans laquelle

- OE est un groupe de formule $-CH_2-CH_2-O-$
- OP est un groupe de formule $-CH_2-CHCH_3-O-$ ou $-CHCH_3-CH_2-O-$,
- X' est un atome d'hydrogène ou un groupe de hydrocarboné,
- s est un nombre moyen supérieur à 1, et
- t est un nombre moyen supérieur ou égal à 0,

Le groupe X est de préférence un groupe de formule $-CH_2-CH_2-CH_2-O-[OE]_q-[OP]_r-X'$, dans laquelle:

- $q \geq 5$,
- $r \geq 0$, et
- X' est un atome d'hydrogène.

Le groupe Y présente avantageusement la formule (II) suivante:

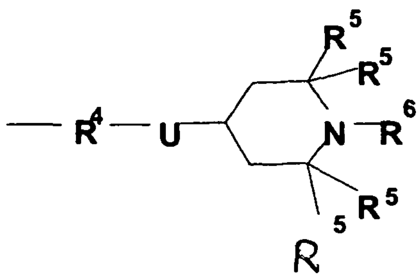

(II)

dans laquelle:

- $R^4$, identique ou différent, est un divalent hydrocarboné divalent choisi parmi:
  - les groupes alkylènes linéaires ou ramifiés, ayant 2 à 18 atomes de carbone;
  - les groupes alkylène-carbonyle dont la partie alkylène, linéaire ou ramifiée, comprend 2 à 20 atomes de carbone;
  - les groupes alkylène-cyclohexylène dont la partie alkylène linéaire ou ramifiée, comprend 2 à 12 atomes de carbone et la partie cyclo-hexylène comporte un groupe -OH et éventuellement 1 ou 2 groupes alkyles ayant 1 à 4 atomes de carbone;
  - les groupes de formule $-R^7-O-R^7$ dans laquelle les groupes $R^7$, identiques ou différents, représentent des groupes alkylènes ayant 1 à 12 atomes de carbone;
  - les groupes de formule $-R^7-O-R^7$ dans laquelle les groupes $R^7$ ont les significations indiquées précédemment et l'un d'entre eux ou les deux sont substitués par un ou deux groupe(s) -OH;
  - les groupes de formule $-R^7-COO-R^7$ dans laquelle les groupes $R^7$ ont les significations indiquées précédemment; et
  - les groupes de formule $-R^8-O-R^9-O-CO-R^8$ dans laquelle les groupes $R^8$ et $R^9$, identiques ou différents, représentent des groupes alkylènes ayant 2 à 12 atomes de carbone et le groupe $R^9$ est éventuellement substitué par un groupe hydroxyle;

- U représente -O- ou $-NR^{10}-$, $R^{10}$ étant un atome d'hydrogène, un groupe alkyle linéaire ou ramifié comprenant 1 à 6 atomes de carbone;
- $R^5$, identique ou différent, est un groupe alkyle linéaire ou ramifié ayant 1 à 3 atomes de carbone ou un groupe phényle; et
- $R^6$ représente un atome d'hydrogène, un groupe $R^5$ ou un radical libre O°.

Le groupe Y est de préférence un groupe de formule (III) suivante:

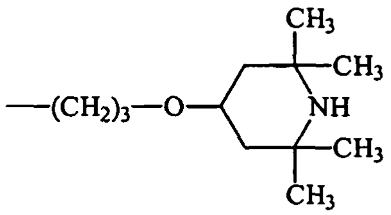 (III)

Le groupe R, identique ou différent, est avantageusement un groupe méthyle, éthyle, isopropyle, tertiobutyle, n-hexyle, octyle, trifluoropropyle, ou phényle. Le groupe R est de préférence un groupe méthyle.
Le polyorganosiloxane de formule (I) est avantageusement un polyorganosiloxane linéaire. Il présente avantageusement la formule (IV) suivante:

$$A\text{-}SiR_2\text{-}O\text{-}[SiRXO]_x\text{-}[SiRYO]_n\text{-}[SiR_2O]_m\text{-}O\text{-}SiR_2\text{-}A \qquad (IV)$$

dans laquelle:

- R, identique ou différent, est un groupe tel que décrit plus haut,
- X, identique ou différent, est un groupe tel que décrit plus haut,
- Y, identique ou différent, est un groupe tel que décrit plus haut,
- A, identique ou différent, est un groupe R, Y, ou X,
- x est un nombre moyen supérieur à 0,
- n est un nombre moyen supérieur à 0,
- m est un nombre moyen supérieur à 0, et
- x+m+n+2 = N.

De préférence:

- A est un groupe R, et
- R est un groupe méthyle.

De préférence:

- m ≥ 10, de préférence m≥ 100
- x ≥ 1, et
- n ≥ 1, encore plus préférablement n≥3.

Le polyorganosiloxane de formule (I) comprend avantageusement entre 0,1 et 0,5 % en poids d'atomes d'azote, par rapport au poids de polyorganosiloxane. Ce taux peut être calculé à partir de la mesure en moles de la quantité d'acide nécessaire pour neutraliser le polyorganosiloxane.
De préférence:

- m ≥ 50, et
- 0,001 ≤ n/(m+n+x) ≤ 0,5, de préférence 0,005 ≤ n/(m+n+x) ≤ 0,1.

Encore plus préférentiellement:

- m ≤ 50, et
- 0,001 ≤ x/(m+n+x) ≤ 0,5, de préférence 0,01 ≤ x/(m+n+x) ≤ 0,2.

Le polyorganosiloxane de formule (I) présente avantageusement une viscosité comprise entre 1000 et 1000000 mPa.s, de préférence entre 5000 et 500000 mPa.s, de préférence entre 10000 et 100000 mPa.s. La viscosité peut être mesurée à une température de 25°C à l'aide d'un viscosimètre de type Brookfield, par exemple à un cisaillement correspondant à 10 tours/min, «spindle 4».
Le polyorganosiloxane de formule (I) peut être préparé par tout procédé adapté. Des procédés utiles sont décrits dans US 5,721,297. On cite notamment les procédés comprenant un greffage du groupe Y par une réaction

d'hydrosilylation. On cite également les procédés de redistribution ou re-arrangement à partir de polyorganosiloxanes ne comprenant pas le groupe Y, et/ou de polyorganosiloxanes comprenant le groupe X, et de polyorganosiloxanes comprenant des groupes Y attachés à des atomes de silicium.

Par exemple, un procédé adapté de redistribution ou re-arrangement peut comprendre les étapes suivantes:

1. Mettre en présence un polydiméthylsiloxane, de préférence cyclique, de l'hexamethyldisiloxane, un copolymère de diméthylsiloxane et de methyl-polyoxyde d'alkylène, et un polyméthylsiloxane cyclique comprenant des groupes Y attachés à de atomes de silicium,
2. Chauffer, introduire un catalyseur basique, par exemple une base forte, et laisser réagir,
3. neutraliser, par exemple à l'aide d'au acide faible, puis isoler un polyorganosiloxane de formule (I), par exemple par dévolatilisation, refroidissement et soutirage.

On précise que le polyorganosiloxane de formule (I) peut être en réalité une composition complexe, comprenant en plus du polyorganosiloxane de formule (I) des quantités plus ou moins importantes de composés utilisés pour sa préparation ou de sous-produits. En outre, le polyorganosiloxane de formule (I) peut comprendre une quantité plus ou moins importante de polyoxyalkylène libre, par exemple polyoxyde d'éthylène et/ou de propylène et/ou de copolymère polyoxyde d'éthylènelpropylène.

Vecteur cosmétiquement acceptable

Tout vecteur cosmétiquement acceptable permettant de formuler le polyorganosiloxane de formule (I), et d'obtenir la forme de composition cosmétique désirée, pour l'utilisation visée, peut être utilisé. Différents vecteurs cosmétiquement acceptables pour différents types de formulations sont connus de l'homme du métier.

A titre d'exemples de vecteurs cosmétiquement acceptables, on peut citer les vecteurs aqueux (comprenant de l'eau), les vecteurs alcooliques (comprenant un alcool, par exemple de l'éthanol, l'isopropanol, l'éthylène glycol ou les polyéthylène glycol), le propylène glycol, les vecteurs hydro-alcooliques (comprenant un mélange d'eau et d'un alcool par exemple de l'éthanol, l'isopropanol, l'éthylène glycol ou les polyéthylène glycol). Certaines huiles, volatiles ou non, peuvent également être utilisées. On cite par exemple les silicones fluides, tels que le cyclopentasiloxane, par exemple le Mirasil CM5 commercialisé par Rhodia.

L'homme du métier sait choisir les vecteurs adaptés aux types de formulations souhaités, et aux utilisations visées. Par exemple des vecteurs aqueux sont généralement utilisés pour des shampoings ou gels-douche. Un vecteur propylène glycol peut être utilisé des compositions sous forme de crèmes. Un vecteur cyclométhicone peut être utilisé pour des compositions de maquillage, par exemple pour des fonds de teint.

Tensioactifs

La composition peut comprendre au moins un tensioactif. Il peut s'agir d'un mélange de différents tensioactifs. Il s'agit de préférence d'au moins un tensioactif anionique, seul ou en mélange. Le tensioactif peut en outre comprendre des tensioactifs anioniques, des tensioactifs amphotères (amphotères vrais ou zwitterioniques), des tensioactifs neutres et/ou des tensioactifs cationiques, seuls ou en mélange. Les compositions comprenant au moins un tensioactif amphotère et éventuellement un tensioactif anionique sont particulièrement avantageuses, notamment pour des raisons de douceur. La teneur totale en tensioactif dans la composition peut être comprise entre 5 et 30% en poids.

Pour des compositions destinées au traitement des cheveux, comme des shampoings, la teneur en tensioactif est avantageusement comprise entre 10 et 20% en poids. De telles compositions peuvent comprendre des sels, par exemple du chlorure de sodium ou d'ammonium, avantageusement en teneur inférieure à 3% en poids.

Pour des compositions destinées au traitement de la peau, comme des gel-douche, la teneur en tensioactif est avantageusement comprise entre 5 et 15% en poids. De telles compositions comprennent également de préférence au moins 2% en poids de sels, par exemple du chlorure de sodium ou d'ammonium.

La proportion en poids de tensioactif anionique par rapport à l'ensemble des tensioactifs est de préférence supérieure à 50%, de préférence supérieure à 70%.

Les tensioactifs anioniques peuvent être choisis parmi les tensioactifs suivants:

- les alkylesters sulfonates, par exemple de formule $R-CH(SO_3M)-CH_2COOR'$, ou les alkylesters sulfates, par exemple de formule $R-CH(OSO_3M)-CH_2COOR'$, où R représente un radical alkyle en $C_8$-$C_{20}$, de préférence en $C_{10}$-$C_{16}$, R' un radical alkyle en $C_1$-$C_6$, de préférence en $C_1$-$C_3$ et M un cation alcalino-terreux, par exemple sodium, ou le cation ammonium. On peut citer tout particulièrement les méthyl ester sulfonates dont le radical R est en $C_{14}$-$C_{16}$;
- les alkylbenzènesulfonates, plus particulièrement en $C_9$-$C_{20}$, les alkylsulfonates primaires ou secondaires, notamment en $C_8$-$C_{22}$, les alkylglycérol sulfonates ;

- les alkylsulfates par exemple de formule $ROSO_3M$, où R représente un radical alkyle ou hydroxyalkyle en $C_{10}$-$C_{24}$, de préférence en $C_{12}$-$C_{20}$ ; M un cation de même définition que ci-dessus ;
- les alkyléthersulfates par exemple de formule $RO(OA)_nSO_3M$ où R représente un radical alkyle ou hydroxyalkyle en $C_{10}$-$C_{24}$, de préférence en $C_{12}$-$C_{20}$ ; OA représentant un groupement éthoxylé et/ou propoxylé ; M représentant un cation de même définition que ci-dessus, n variant généralement de 1 à 4, comme par exemple le lauryléthersulfate avec n = 2 ;
- les alkylamides sulfates, par exemple de formule RCONHR'$OSO_3M$ où R représente un radical alkyle en $C_2$-$C_{22}$, de préférence en $C_6$-$C_{20}$, R' un radical alkyle en $C_2$-$C_3$, M représentant un cation de même définition que ci-dessus, ainsi que leurs dérivés polyalcoxylés (éthoxylés et/ou propoxylés) (alkylamidoether sulfates
- les sels d'acides gras saturés ou insaturés, par exemple comme ceux en $C_8$-$C_{24}$, de préférence en $C_{14}$-$C_{20}$ et d'un cation alcalino-terreux, les N-acyl N-alkyltaurates, les alkyliséthionates, les alkylsuccinamates et alkylsulfo succinates, les monoesters ou diesters de sulfosuccinates, les N-acyl sarcosinates, les polyéthoxycarboxylates
- les mono et di esters phosphates, par exemple de formule suivante :

  $(RO)_x$-P(=O)$(OM)_x$ ou R représente un radical alkyle, alkylaryle, arylalkyle, aryle, éventuellement polyalcoxylés, x et x' étant égaux à 1 ou 2, à la condition que la somme de x et x' soit égale à 3, M représentant un cation alcalino-terreux ;

Les <u>tensioactifs non ioniques</u> peuvent être choisis parmi les tensioactifs suivants:

- les alcools gras alcoxylés ;
- les triglycérides alcoxylés
- les acides gras alcoxylés
- les esters de sorbitan alcoxylés
- les amines grasses alcoxylées
- les di(phényl-1 éthyl) phénols alcoxylés
- les tri(phényl-1 éthyl) phénols alcoxylés
- les alkyls phénols alcoxylés
- les produits résultant de la condensation de l'oxyde d'éthylène avec un composé hydrophobe résultant de la condensation de l'oxyde de propylène avec le propylène glycol, tels les Pluronic commercialisés par BASF ;
- les produits résultant de la condensation de l'oxyde d'éthylène le composé résultant de la condensation de l'oxyde de propylène avec l'éthylènediamine, tels les Tetronic commercialisés par BASF ;
- les alkylpolyglycosides comme ceux décrits dans US 4565647 ;
- les amides d'acides gras par exemple en $C_8$-$C_{20}$ ;

Les <u>tensioactifs amphotères</u> (amphotères vrais comprenant un groupe ioniques et un groupe potentiellement ionique de charge opposée, ou zwitterioniques comprenant simultanément deux charges opposées) peuvent être choisis parmi les tensioactifs suivants:

- les bétaïnes de manière générale, notamment carboxybétaïnes de par exemple la lauryl bétaïne (Mirataine BB de la société Rhodia) ou l'octylbétaïne; les amidoalkylbétaïnes, comme la cocamidopropyl bétaïne (CAPB) (Mirataine BDJ de la société Rhodia Chimie) ;
- les sulfo-bétaïnes ou sultaines comme la cocamidopropyl hydroxy sultaïne (Mirataine CBS de la société Rhodia) ;
- les alkylamphoacétates et alkylamphodiacétates, comme par exemple comprenant une chaîne coco ou lauryle (Miranol C2M, C32, L32 notamment, de la société Rhodia) ;
- les alkylamphopropionates ou les alkylamphodipropionates, (Miranol C2M SF) ;
- les alkyl amphohydroxypropyl sultaïnes (Miranol CS).

Les <u>tensioactifs cationiques</u> peuvent être choisis parmi les sels d'amines grasses primaires, secondaires ou tertiaires, éventuellement polyethoxylées, les sels d'ammoniums quaternaires tels que les chlorures ou les bromures de tetraalkylammonium, d'alkylamidoalkylammonium, de trialkylbenzylammonium, de trialkylhydroxyalkylammonium, ou d'alkylpyridinium, les dérivés d'imidazoline, les oxydes d'amines à caractère cationique.

<u>Agent de stabilisation et/ou de conditionnement et/ou d'aide au conditionnement</u>

La composition cosmétique selon l'invention peut avantageusement comprendre au moins un agent de stabilisation et/ou de conditionnement (agents conditionneurs) et/ou d'aide au conditionnement. On parle aussi parfois d'agents de suspension. Par aide au conditionnement, on entend que la présence de l'agent améliore le conditionnement

lié à d'autre composés, par exemple des huiles ou silicones. Les agents sont entendus comme des agents différents du polyorganosiloxane de formule (I). De tels agents sont connus de l'homme du métier. La composition selon l'invention peut comprendre plusieurs de ces agents (mélanges ou combinaisons), pour associer leurs effets et/ou créer des synergies. Par ailleurs, certains agents peuvent exercer plusieurs fonctions. C'est le cas par exemple des polysaccharides, et leurs dérivés cationiques, par exemple des dérivés cationiques de guars.

La proportion en poids de tels agents peut être typiquement de 0,1% à 10% en poids, de préférence de 0,3% à 8% en poids, pour des polysaccharides ou d'autres agents.

A titre d'exemples d'agents de stabilisation (ou agents stabilisants), on peut citer:

- les polyacrylates réticulés, par exemple les polymères de type CARBOPOL ou CARBOMER commercialisés par BF Goodrich ou Noveon, ACRITAMER commercialisés par RITA ou TEGO CARBOMER commercialisés par Goldschmidt. Ces composés peuvent être typiquement présents en quantité de 0,1 à 3%, de préférence de 0,3 à 2%, en poids par rapport à la composition.
- les copolymères des acrylates/aminoacrylates/ itaconates PEG 20 alkyles $C_{10}$-$C_{30}$ commercialisés par National Starch sous le nom STRUCTURE PLUS. Ces composés peuvent être typiquement présents en quantité de 0,1 à 3%, de préférence de 0,3 à 2%, en poids par rapport à la composition.
- les solides insolubles formant un réseau dans la composition. Il peut s'agir de mono et/ou di-esters d'acides gras d'éthylène glycol, les acides gras étant de préférence en $C_{16}$-$C_{18}$. Il peut en particulier s'agir d'éthylène glycol distéarate (EGDS), par exemple commercialisé par Rhodia en concentré avec d'autres ingrédient sous le nom MIRASHEEN. Ce composé peut être typiquement présent en quantité de 3 à 10%, de préférence de 5 à 8% en poids par rapport à la composition.

On peut citer également des agents viscosants, gelifiants ou texturants comme les copolymères acryliques anioniques de type ACULYNE commercialisés par ISP ou Rohm & Haas), les polysaccharides et leurs dérivés non cationiques tels que les dérivés de la cellulose comme l'hydroxypropylcellulose, la carboxyméthylcellulose, les dérivés non-ioniques de guars comme l'hydroxypropyl guar (par exemple les Jaguar HP commercialisé par Rhodia), la caroube, la gomme de tara ou de cassia, la gomme xanthane (par exemple les Rhodicare commercialisés par Rhodia), les succinoglycanes (par exemple le Rheozan commercialisé par Rhodia), les alginates, les carraghénannes, les dérivés de la chitine ou tout autre polysaccharide à fonction texturante. Ces polysaccharides et leurs dérivés peuvent être incorporés seuls ou en combinaison synergique à d'autres polysaccharides. Ces composés peuvent être typiquement présents en quantité de 0,1 à 3%, de préférence de 0,3 à 1%, en poids par rapport à la composition.

A titre d'exemples d'agents de stabilisation et/ou d'agents de conditionnement et/ou d'aide ou conditionnement, on peut citer:

- les polymères cationiques dérivés de polysaccharides, par exemple les dérivés cationiques de celluloses, les dérivés cationiques d'amidons, les dérivés cationiques de guars, les dérivés cationique de caroube.
- les polymères cationiques synthétiques,
- les mélanges ou combinaisons de ces agents.

Les polymères cationiques, synthétiques ou non, pouvant assurer une fonction d'agent de conditionnement, sont notamment des polymères de type polyquaternium, comme par exemple les polyquaternium-1, polyquatemium-2, polyquaternium-4, polyquaternium-5, polyquaternium-6 (également connu comme Merquat 1000 disponible auprès de Nalco), polyquaternium-7 (également connu comme Merquat 5500 disponible auprès de Nalco), polyquaternium-8, polyquaternium-9, polyquaternium 10 (également connu comme Polymer JR 400, commercialisé par Amercol), polyquaternium-11, polyquaternium-12, polyquaternium-13, polyquaternium-14, polyquaternium-15, polyquaternium-16, polyquaternium-17, polyquatemium-18, polyquaternium-19, polyquaternium-20, polyquaternium-22 (également connu comme Merquat 280, 281, 298 disponibles auprès de Nalco), polyquaternium-24, polyquaternium-27, polyquaternium-28, polyquaternium-29 (également connu comme Kytamer KCO disponible auprès de Amerchol), polyquaternium-30, polyquaternium-31, polyquaternium-32, polyquaternium-33, polyquaternium-34, polyquaternium-35, polyquaternium-36, polyquaternium-37, polyquaternium-39 (également connu comme Merquat 3300, 3331 disponibles auprès de Nalco), polyquaternium-44, polyquaternium-27 (également connu comme Merquat 2001 disponible auprès de Nalco) et polyquaternium-55.

Les dérivés cationiques des guars peuvent avoir une fonction d'agent de stabilisation des formulations, d'agent de conditionnement et/ou d'agent d'aide au conditionnement. A titre d'exemples on peut citer:

- le guar hydroxypropyl trimonium chlorure, (Jaguar C 13S, Jaguar C14S, Jaguar C17, Jaguar Excel, Jaguar C 2000, commercialisés par RHODIA)
- l'hydroxypropyl guar hydroxypropyl trimonium chlorure (Jaguar C162, commercialisés par RHODIA),

- l'éther de poly (oxyéthanediyl-1, 2) hydroxy-2 chlorure de triméthylammonium-3 propyl cellulose ou polyquatemium-10.

Autre polyorganosiloxane

La composition selon l'invention peut comprendre un polyorganosiloxane différent du polyorganosiloxane de formule (I). Cet autre polyorganosiloxane peut être par exemple présent sous forme d'une émulsion, définissant dans le vecteur cosmétiquement acceptable une deuxième famille de gouttelettes (on parle souvent de co-émulsions). Il peut également être en mélange avec le polyorganosiloxane de formule (I), sous forme d'une émulsion de gouttelettes du mélange (on peut parler d'émulsions de mélanges), ou sous forme de gouttelettes dispersées dans le polyorganosiloxane de formule (I) (on peut parler d'émulsions multiples).

Le polyorganosiloxane différent du polyorganosiloxane de formule (I) peut être un polyorganosiloxane comprenant des groupes polaires, ou un polyorganosiloxane non polaire.

A titre d'exemples de polyorganosiloxanes comprenant des groupes polaires, on peut citer: les dimethiconols, amodimeticones, trimethylsilyl amodimethicone, dimethicone copolyols, temary copolyols, Silatrizole, dimethicone copolyol amine, silicone quaternium (CTFA silicone quaternium 1 à 10).

A titre d'exemples de polyorganosiloxanes non polaires, on peut citer les polydimethylorganosiloxanes (PDMS ou diméthicone), les silicones présentant des groupes phenyl, les silsesquioxane (structure «T») & silicates (structure «Q»), les silicones réticulées, les copolymères comprenant des groupes silicones, les résines silicones, les cires silicones, les siloxanes alkyle methyle volatiles.

De même que pour le polyorganosiloxane de formule (I), les émulsions de polyorganosiloxane différent du polyorganosiloxane de formule (I) peuvent être préparées par émulsification in situ ou par émulsification préalable, et présenter des tailles de gouttelettes inférieures à 0,15 $\mu$m, ou comprises entre 0,15$\mu$m et 2 $\mu$m, ou supérieures ou égales à 2$\mu$m. On se réfère au passage consacré aux émulsions ci-après.

Filtres UV

La composition selon l'invention peut comprendre des agents de filtres UV. Il peut s'agir de d'agents organiques, ou minéraux. Il peut par exemple s'agir d'agents minéraux tels que des dispersions de particules à base de dioxyde de titane, d'oxyde de zinc, ou d'oxyde de cérium, de préférence sous forme nanoparticulaire, le cas échéant recouvertes d'une couche à base d'oxyde ou hydroxyde de silice ou d'aluminium, par exemple la dispersion commercialisée sous la dénomination Mirasun® TiW60 par Rhodia. Il peut également s'agir de molécules organiques. De telles molécules sont connues de l'homme du métier. A titre d'exemples de molécules organiques, on cites les composés suivants: Eusolex OCR ou Eusolex 6300 (Merck); Parsol 1789, Parsol HS, ou Parsol MCX (Givaudan Roure); Mexoril SX (Chimex); Escalol 567, Escalol 587, ou Escalol 507 (ISP/Van Dyk); Uvinul MS-40, Uvinul T-150, ou Spectrasorb UV-24 (BASF); Neo Heliopan MA ou Neo Heliopan Type E 1000 (Haarmann & Reimer); Tinosorb M (Ciba), Homomenthyl salicylate.

Autres ingrédients

A titre d'autres ingrédients pouvant être compris dans la composition, on peut citer des agents de coloration, teintures ou colorants, des fragrances, des parfums, des agents masquant les fragrances, des polymères, des agents tampons, des complexants, des capsules complexantes, des sels solubles, par exemple des sels de métaux, d'alcalins, d'alcalino-terreux, ou d'ammoniums, par exemple NaCl ou $NaSO_4$ ou $NH_4Cl$, des acides de Lewis, des épaississants particulaires, des épaississants polymériques, des cires épaississantes, des huiles, des agents émollients, des humectants, des agents hydratants, des agents nacrants, des opacifiants, des agents dispersants, des agents favorisant la suspension de particules, des agent anti-microbiens, des conservateurs, des protéïnes, des extraits végétaux, des oxydants, des agents modifiant la viscosité, des agents de gélification, des chélatants, des réducteurs.

La composition peut en outre comprendre une grande variété d'agents actifs, hydrophiles ou non. Il peut s'agir d'agents antifongiques, anti-bactériens, par exemple le triclosan, d'agents anti-pelliculaires, par exemple du zinc-pyrithione, d'agents anti-vieillissement, d'agents anti-cellutite.

A titre d'exemples de matières actives utilisables dans le domaine de la cosmétique on peut citer les vitamines, comme la vitamine A et ses dérivés notamment ses esters comme l'acétate, le palmitate, le propionate, la vitamine B2, l'acide pantothénique, la vitamine D et la vitamine E ; les mono-, di- et triglycérides ; les bactéricides ; les agents absorbeurs d'UV, comme les dérivés aminobenzoate de type PABA et PARA, les salicylates, les cinnamates, les anthranilates, les dibenzoylméthanes, les dérivés du camphre et leurs mélanges.

Les agents anti-vieillissement peuvent de même être utilisés. A titre d'exemples de tels agents on peut citer notamment les rétinoïdes, les vitamines liposolubles, les dérivés de la vitamine C comme les esters notamment l'acétate, le propionate, le palmitate ; les céramides, les pseudo-céramides, les phospholipides, les acides gras, les alcools gras,

le cholestérol, les stérols et leurs mélanges. Comme acides gras et alcools préférés, on peut plus particulièrement citer ceux possédant des chaînes alkyles, linéaires ou ramifiées contenant de 12 à 20 atomes de carbone. Il peut notamment s'agir d'acide linoléique.

On peut de même mettre en oeuvre des agents anti-cellulite, tels que notamment l'isobutylméthylxanthine et la théophyline ; ainsi que des agents anti-acné, comme par exemple le résorcinol, l'acétate de résorcinol, le peroxyde benzoyle et de nombreux composés naturels.

Les arômes, parfums, huiles essentielles, essences, peuvent aussi être utilisés en tant que matière active. A titre d'exemple, on peut citer les huiles et/ou essences de menthe, de menthe verte, de menthe poivrée, de menthol, de vanille, de cannelle, de laurier, d'anis, d'eucalyptus, de thym, de sauge, de feuille de cèdre, de noix de muscade, de citrus (citron, citron vert, pamplemousse, orange), de fruits (pomme, poire, pêche, cerise, prune, fraise, framboise, abricot, ananas, raisin, etc.), seules ou en mélanges. On peut aussi mettre en oeuvre des composés comme le benzaldéhyde, l'acétate d'isoamyle, le butyrate d'éthyle, etc.

Les agents anti-microbiens peuvent être choisis parmi le thymol, le menthol, le triclosan, le 4-hexylrésorcinol, le phénol, l'eucalyptol, l'acide benzoïque, le peroxyde benzoïque, le parabène de butyle, et leurs mélanges.

Les compositions cosmétiques peuvent également contenir des polymères présentant des propriétés filmogènes pouvant être utilisés pour apporter une fonction fixante. Ces polymères sont généralement présents à des concentrations comprises entre 0,01 et 10 %, préférentiellement entre 0,5 et 5 %. Ils sont préférentiellement du type polyvinylpyrrolidone, copolymères de polyvinylpyrrolidone et de méthyl méthacrylate, copolymère de polyvinylpyrrolidone et d'acétate de vinyle, copolymères polytéréphtalate d'éthylène glycol/polyéthylène glycol, polymères copolyesters téréphtaliques sulfonés.

On peut également incorporer aux compositions cosmétiques des agents hydratants. A titre illustratif de ces derniers, on peut notamment citer le glycérol, le propylène glycol, l'urée, le collagène, la gélatine, et des émollients qui sont généralement choisis parmi les alkylmonoglycérides, les alkyldiglycérides, les triglycérides comme les huiles extraites des plantes et des végétaux ou leurs dérivés hydrogénés, les huiles minérales ou les huiles paraffiniques, les diols, les esters gras, les silicones (voir plus haut).

Des agents conservateurs comme les esters de l'acide *p*-hydroxybenzoïque, le benzoate de sodium, ou tout agent chimique évitant la prolifération bactérienne ou des moisissures et utilisé traditionnellement dans les compositions cosmétiques sont généralement introduits dans ces compositions à hauteur de 0,01 à 3 % en poids. Des agents conservateurs sont par exemple commercialisés sous les noms Glydant, Germaben, Kathon.

<u>Forme physico-chimique de la composition cosmétique</u>

La composition cosmétique comprend un vecteur cosmétiquement acceptable et le polyorganosiloxane de formule (I). Le polyorganosiloxane est dispersé dans le vecteur cosmétiquement acceptable, ou dans un mélange d'ingrédients comprenant le vecteur cosmétiquement acceptable. La dispersion peut par exemple être:

- une solution du polyorganosiloxane de formule (I) dans le vecteur cosmétiquement acceptable ou dans un mélange comprenant le vecteur cosmétiquement acceptable;
- une émulsion stable de gouttelettes comprenant le polyorganosiloxane de formule (I) dans le vecteur cosmétiquement acceptable,
- une émulsion inverse dans laquelle le polyorganosiloxane de formule (I) est compris dans la phase externe, ou
- une association de phases séparées en au moins une strate comprenant le vecteur cosmétiquement acceptable, et un strate comprenant le polyorganosiloxane de formule (I), pouvant former une dispersion de gouttelettes comprenant le polyorganosiloxane de formule (I) dans le vecteur cosmétiquement acceptable après agitation par l'utilisateur.

Bien entendu, le vecteur cosmétiquement acceptable peut comprendre d'autres ingrédients que le polyorganosiloxane de formule (I), ces autres ingrédients pouvant être présents en solution ou en dispersion, par exemple sous forme d'une suspension de particules solides, ou d'une émulsion définissant une famille de gouttelettes.

De même, le polyorganosiloxane de formule (I) peut définir une phase dans laquelle sont dispersés un ou plusieurs autres ingrédients.

On détaille ci-dessous quelques caractéristiques de compositions cométiques selon l'invention sous forme d'émulsions.

<u>Emulsions</u>

Les compositions cosmétiques selon l'invention peuvent être sous forme d'émulsions de gouttelettes comprenant le polyorganosiloxane de formule (I) dispersées dans le vecteur cosmétiquement acceptable, de préférence dans

un vecteur aqueux.

Les gouttelettes de l'émulsion peuvent être de taille plus ou moins importante. On peut ainsi se référer à des microémulsions, à des mini-émulsions, ou à des macroémulsions. Dans la présente demande, le terme «émulsion» couvre notamment tous ces types d'émulsions. Sans vouloir être lié à une quelconque théorie, on précise que les microémulsions sont généralement des systèmes thermodynamiquement stable, comprenant généralement d'importantes quantités d'agents d'émulsification. Les autres émulsions sont généralement des systèmes en état non-thermodynamiquement stable, conservant pendant un certain temps, en état métastable l'énergie mécanique fournie lors de l'émulsification. Ces systèmes comprennent généralement des quantités moindres d'agents d'émulsification.

Les compositions sous forme d'émulsions peuvent être obtenues par mélange du vecteur, de préférence aqueux, du polyorganosiloxane de formule (1), et en général d'un agent d'émulsification, puis émulsification. On peut parler d'émulsification in situ.

Les compositions sous forme d'émulsion peuvent également être obtenues par mélange du vecteur, de préférence aqueux, avec une émulsion préalablement préparée de gouttelettes comprenant le polyorganosiloxane de formule (I) dans une phase externe, de préférence miscible avec le vecteur cosmétiquement acceptable, de préférence de même nature que ledit vecteur, de préférence un vecteur aqueux. On peut préférer ce mode de réalisation car il est simple à mettre en oeuvre. En outre ce mode de réalisation est particulièrement adapté pour la mise en oeuvre de compositions cosmétiques dans lesquelles le polyorganosiloxane de formule (I) est sous forme de microémulsion. On peut parler d'émulsification préalable.

Selon un mode particulier de réalisation, l'émulsion est une microémulsion, dont la taille des gouttelettes est inférieure à 0,15 μm. Dans ce mode de réalisation, la composition comprend de préférence une proportion supérieure à 10% en poids, de préférence au moins 15% en poids d'agent d'émulsification par rapport au poids de polyorganosiloxane de formule (I).

La taille des gouttelettes de microémulsion peut être mesurée sur une émulsion préparée préalablement à son introduction dans la composition cosmétique, par diffusion de Lumière Dynamique (DQEL), par exemple comme décrit ci-après. L'appareillage utilisé est par exemple constitué d'un laser Spectra-Physics 2020, d'un corrélateur Brookhaven 2030 et de l'informatique associée. L'échantillon étant concentré, il est dilué dans l'eau désionisée et filtré à 0,22 μm, pour être en final à 2% en poids. Le diamètre obtenu est un diamètre apparent. Les mesures sont faites à 90° et 135° d'angle. Pour les mesures de taille, outre l'analyse classique par les cumulants, trois exploitations de la fonction d'autocorrélation sont utilisées (l'échantillonnage exponentiel ou EXPSAM décrit par le Pr. Pike, la méthode « Non Negatively Constrained Least Squares » ou NNLS et la méthode CONTIN décrite par le Pr. Provencher), qui donnent chacune une répartition de taille pondérée par l'intensité diffusée, et non pas par la masse ou le nombre. Il est tenu compte de l'indice de réfraction et de la viscosité de l'eau.

Selon un mode avantageux, la microémulsion est transparente. La microémulsion peut par exemple présenter une transmittance d'au moins 90%, de préférence d'au moins 95%, à une longueur d'onde de 600 nm, mesurée par exemple à l'aide d'un spectromètre Lambda 40 UV-Vis, à une concentration à 0.5% en poids dans l'eau. Dans ce cadre, la composition cosmétique peut avantageusement être transparente. Elle peut par exemple présenter une transmittance d'au moins 90%, de préférence d'au moins 95%, à une longueur d'onde de 600 nm, mesurée par exemple à l'aide d'un spectromètre Lambda 40 UV-Vis.

Selon un autre mode particulier de réalisation, l'émulsion est une émulsion dont la taille moyenne des gouttelettes est supérieure ou égale à 0,15 μm, par exemple supérieure à 0,5 μm, ou à 1 μm, ou à 2 μm, ou à 10 μm, ou à 20 μm, et de préférence inférieure à 100 μm. La taille des gouttelettes peut être mesurée sur une émulsion préparée préalablement à son introduction dans la composition cosmétique, ou directement sur la composition cosmétique diluée dans de l'eau, par microscopie optique et/ou granulomètrie laser (Horiba LA-91 0 laser scattering analyzer). Dans ce mode de réalisation, la composition comprend de préférence une proportion inférieure à 10% en poids d'agent d'émulsification, par rapport au poids de polyorganosiloxane de formule (I).

Comme mentionné ci-dessus les gouttelettes de l'émulsion peuvent comprendre d'autres ingrédients que le polyorganosiloxane de formule (I). Ainsi, le polyorganosiloxane de formule (I) peut être mélangé à un ingrédient miscible, par exemple une huile, de préférence une huile silicone, le mélange formant l'émulsion. Les gouttelettes de polyorganosiloxane de formule (I) peuvent également comprendre une émulsion de gouttelettes de plus petite taille d'une phase non miscible (phase interne). L'émulsion est alors une émulsion multiple comprenant une phase interne dispersée dans une phase intermédiaire comprenant le polyorganosiloxane de formule (I), elle-même dispersée dans le vecteur. Les ingrédients pouvant être compris dans la phase interne peuvent par exemple être des ingrédients actifs procurant un effet positif sur la peaux et/ou les cheveux. Il peut s'agir également d'agents favorisant le dépôt du polyorganosiloxane de formule (I), ou d'autres ingrédients, sur la peaux et/ou les cheveux.

Agents d'émulsification

Les agents d'émulsification sont des agents pouvant permettre d'obtenir une émulsion du polyorganosiloxane

de formule (I) dans le vecteur, de préférence de l'eau. Il peut par exemple s'agir:

- d'un tensioactif non ionique,
- d'un polymère amphiphile non ionique, éventuellement associé à un ou plusieurs tensioactifs anioniques et/ou polymères amphiphiles anioniques,
- d'un tensioactif particulaire éventuellement associé à un co-tensioactif, ou
- d'un colloïde protecteur.

Tensioactif particulaire

Selon un mode particulier, l'agent d'émulsification est un tensioactif particulaire éventuellement associé à un co-tensioactif.
Le tensioactif particulaire est de préférence choisi parmi les composés particulaires solides, dont l'angle de contact est proche de 0°, associés à au moins un co-stabilisants choisi parmi les tensioactifs non-ioniques, anioniques, cationiques ou zwitterioniques.
Le tensioactif particulaire est par exemple une silice de précipitation, une silice colloïdale, un silicoaluminate, de l'oxyde de zinc, de l'oxyde de titane, ou un mélange de ces composés, ces composés comprenant le cas échéant un traitement de surface.

Colloïde protecteur

Selon un autre mode particulier l'agent d'émulsification est un colloïde protecteur. Il peut par exemple s'agir d'un alcool polyvinylique, le cas échéant partiellement hydrolysé.
La teneur en colloïde protecteur est avantageusement de 3 à 30 % en poids sec par rapport à l'émulsion interne, de préférence de 5 à 25%.

Tensioactif non ionique

Selon un autre mode particulier l'agent d'émulsification comprend un tensioactif non ionique. Il s'agit de préférence d'un tensioactif non ionique polyalcoxylé, par exemple choisi parmi:

- les alcools gras alcoxylés
- les triglycérides alcoxylés
- les acides gras alcoxylés
- les esters de sorbitan alcoxylés
- les amines grasses alcoxylées
- les di(phényl-1 éthyl) phénols alcoxylés
- les tri(phényl-1 éthyl) phénols alcoxylés, et
- les alkyls phénols alcoxylés

où le nombre de motifs alcoxy, plus particulièrement oxyéthyléne et/ou oxypropyléne, est tel que la valeur de HLB est supérieure ou égale à 10.

Polymère amphiphile non ionique

Selon un autre mode particulier l'agent d'émulsification externe comprend un polymère amphiphile non ionique. Ce polymère peut être associé à un ou plusieurs tensioactifs anioniques et/ou polymères amphiphiles anioniques.
A titre de polymères amphiphiles non ionique on cite les copolymères triblocs (polyéthylène glycol) - (polypropylène glycol) - (polyéthylène glycol).
Pour ce qui a trait aux polymères amphiphiles non ioniques ou anioniques, on peut mettre en oeuvre un polymère comprenant au moins deux blocs, l'un d'eux étant hydrophile, l'autre hydrophobe. On peut mettre en oeuvre un copolymère peignes.
Lesdits polymères amphiphiles peuvent de manière avantageuse, être obtenus par polymérisation radicalaire dite vivante ou contrôlée. A titre d'exemples non limitatifs de procédés de polymérisation dite vivante ou contrôlée, on peut notamment se référer aux demandes WO 98/58974, WO 00/75207 et WO 01/42312 (xanthate), WO 98/01478 (dithioesters), WO 99/03894 (nitroxydes) ; WO 99/31144 (dithiocarbamates), WO 02/26836 (dithiocarbazates) ; WO 02/10223 (dithiophosphoroesters), WO 96/30421 (polymérisation radicalaire par transfert d'atome - ATRP).
Les polymères amphiphiles peuvent aussi être obtenus par polymérisation anionique.

Ils peuvent de même être préparés en mettant en jeu des polymérisations par ouverture de cycle (notamment anionique), ou par modification chimique du polymère.

Plus particulièrement, en ce qui concerne le polymère amphiphile non ionique, de préférence polyoxyalkyléné, présent de la phase aqueuse externe, il peut être choisi parmi les polymères miscibles au moins en partie dans la phase aqueuse externe et de préférence parmi les copolymères triblocs polyéthylène glycol-polypropylène glycolpolyéthylène glycol.

Il est précisé que des polymères de type alcool polyvinylique ou triblocs polyacide acrylique/polyacrylate de butyle/polyacide acrylique peuvent être utilisés à cette fin.

Type de formulation de la composition et utilisations

La composition selon l'invention peut être formulée sous différentes formes, dépendant de l'aspect que l'on souhaite lui conférer, des propriétés sensorielles (viscosité, toucher, permanence ...) que l'on souhaite lui conférer, et bien entendu de l'utilisation que l'on souhaite en faire. Les différents types de formulations et les différentes utilisations sont modulés par la nature et la quantité des ingrédients présents dans la composition, et sont connus de l'homme du métier.

Ainsi la composition peut être formulée sous forme de gels, de fluides plus ou moins visqueux, de laits, de crèmes, d'huiles, de sprays, de mousses, de gels en bâtonnets (sticks), de pâtes, de lotions, de concentrés de teinture etc...

Les compositions peuvent notamment être choisies parmi les compositions listées dans le tableau I ci-dessous, avec des formes physico-chimiques du polyorganosiloxane de formule (1), des types de formulation et des utilisations également listées dans le tableau (1) ci-dessous. Pour ces compositions, formes physico-chimiques, type de formulations et utilisations, on peut se reporter à des parties plus détaillées de la présente demande.

Tableau 1

| Composition | Forme physico-chimique du polyorganosiloxa ne de formule (I) | Type de formulation | Utilisation |
|---|---|---|---|
| Shampoings | Emulsion | Fluide | Nettoyage et/ou soin des cheveux et/ou coloration temporaire et/ou fixation de la coloration, avec rinçage |
| Après-shampoing | Emulsion | Fluide | Soin des cheveux, et/ou démêlage et/ou aide au coiffage et/ou coloration temporaire et/ou fixation de la coloration et/ou conditionnement et/ou conditionnement après coloration, avec ou sans rinçage |
| Gel-douche | Emulsion | Fluide ou Gel | Nettoyage et/ou soin de la peau |
| Masque capillaire | Emulsion | Fluide très visqueux | Soin des cheveux |
| Crème solaire | Emulsion | Crème | Protection de la peau contre les UV |
| Lait Solaire | Emulsion | Lait | Protection de la peau contre les UV |
| Huile Solaire | Emulsion inverse ou solution | Huile | Protection de la peau contre les UV |
| Spray solaire | Emulsion | Fluide | Protection de la peau contre les UV |

(suite)

| Composition | Forme physico-chimique du polyorganosiloxa ne de formule (I) | Type de formulation | Utilisation |
|---|---|---|---|
| Crème de soin | Emulsion | Crème | Soin de la peau |
| Démaquillant | Emulsion | Crème ou fluide ou gel | Soin et/ou nettoyage de la peau et/ou des cils |
| Maquillage | Emulsion, ou suspension inverse ou solution | Crème, fluide, mascara, poudre, Gels, Bâtonnets | Coloration de la peau ou des cils |
| Déodorant | Emulsion ou Emulsion inverse | Aérosol, Gel, Bâtonnets, substance pouvant être appliquée à l'aide d'un applicateur à boule | Réduction des effets de la transpiration, appliqué sur la peau |
| Mousse à raser | Emulsion | Liquide très fluide ou gel formant une mousse après propulsion aérosol | Préparation au rasage |
| Spray Coiffant ou fixant | Emulsion | Fluide | Mise en forme des cheveux |
| Gel Coiffant ou fixant | Emulsion | Gel | Mise en forme des cheveux |
| Mousse coiffante ou fixante | Emulsion | Liquide très fluide ou gel formant une mousse après propulsion aérosol | Mise en forme des cheveux |
| Composition tinctoriale | Emulsion | Gel ou liquide visqueux | Teinture permanente ou semi-permanente |

Parmi les utilisations des compositions, on peut mentionner les utilisations dans lesquelles la composition est destinée à être rincée et les utilisations dans lesquelles la composition est destinée à ne pas être rincée.

Compositions destinées à être rincées («rinse-off»)

Selon des modes de réalisation intéressants, la composition est une composition pour le soin de la peau et/ou des cheveux, de préférence pour de nettoyage et/ou le traitement et la peau et/ou des cheveux, ladite composition étant sous forme d'un fluide. Il s'agit avantageusement d'un gel douche, d'un shampoing, d'un après-shampoing destiné à être rincé, d'un masque cutané ou capillaire, destiné à être rincé après utilisation.

Pour les gels-douche et shampoings, la composition peut avantageusement comprendre:

- au moins un tensioactif anionique et/ou amphotère, seul ou en mélange,
- éventuellement, au moins un agent de stabilisation et/ou de conditionnement et/ou d'aide au conditionnement, ou un mélange de tels agents,
- éventuellement un autre polyorganosiloxane,
- des mélanges de ces ingrédients.

De tels ingrédients ont été décrits plus haut.

Pour les après-shampoing destinés à être rincés, la composition peut avantageusement être une formulation assez visqueuse, par exemple une crème, sous forme d'une émulsion comprenant une phase aqueuse dans laquelle sont dispersées une phase émulsionnée huileuse texturante, et des gouttelettes émulsionnées du polyorganosiloxane de formule (I). La phase aqueuse comprend avantageusement un agent de conditionnement, par exemple un polymère cationique. De tels polymères ont été décrits plus hauts. La phase aqueuse peut également avantageusement comprendre un tensioactif cationique. De tels tensioactifs ont été décrits plus haut. Il peut s'agir par exemple de chlorure de stearyl benzyl dimethyl ammonium, de chlorure de cetyl trimethyl ammonium (chlorure de cetrimonium), de chlorure de distearyldimethyl ammonium or stearamidopropyldimethylamine, par exemple en quantité 0.3 à 2% en poids.

Compositions destinées à ne pas être rincées («leave-on»)

Selon des modes de réalisation intéressants, la composition est une composition pour le soin de la peau et/ou des cheveux, sous forme d'un fluide ou sous une autre forme, de préférence pour le traitement et/ou la protection et/ou la modification de l'aspect de la peau et/ou des cheveux, destinée à être laissée sur la peau et/ou les cheveux après application.

Il peut par exemple s'agir d'un après-shampoing destiné à ne pas être rincé, d'un lait démêlant, d'une eau démêlante, d'une eau de lissage, d'un revêtement de cuticule («cuticle coat»), d'un produit de soin capillaire coiffant, d'un produit de soin capillaire coiffant et recoiffant, d'un produit de protection solaire (crème solaire, lait solaire, huile solaire), d'une crème de soin, d'un démaquillant, d'un maquillage, de lingettes démaquillantes ou hydratantes, de mousses à raser, de mousses coiffantes ou fixantes, de gels coiffants ou fixants.

Les gels-douche, shampoing ou après-shampoings, destinés à être rincés ou non, comprenant le polyorganosiloxane de formule (I), peuvent ainsi présenter des améliorations en matière de:

- fixation des colorations opérées avant ou pendant l'application de la composition,
- conditionnement des cheveux, particulièrement sur cheveux abîmé et/ou sur les pointes,
- conditionnement de la peau,
- modulation du conditionnement des cheveux et/ou de la peau (conditionnement doux ou important)
- modulation du conditionnement des cheveux et/ou de la peau en fonction du taux d'azote présent dans le polyorganosiloxane,
- effets cosmétiques tels que la douceur, la souplesse, le démêlage, la brillance, l'aptitude au coiffage sur cheveux secs ou mouillés,
- faible jaunissement,
- réparation d'endommagement liés au soleil, à des décolorations liées au soleil ou à d'autres conditions extérieures, ou à une usure,
- préservation et/ou faible dégradation de composés compris dans la composition,
- longue tenue d'un actif délivré sur la peau et/ou les cheveux.

Le polyorganosiloxane de formule (I) peut en particulier être utilisé dans des compositions destinées aux traitement de cheveux avant été exposés ou étant exposés à compositions de teintures comprenant un agent d'oxydation, typiquement des compositions de teinture durable comprenant une base d'oxydation ou des compositions pour la décoloration ou l'éclaircissement des cheveux, comprenant un agent d'oxydation. A ce titre, il peut s'agir d'un shampoing, d'un après shampoing, ou d'une composition de traitement ou conditionnement des cheveux après teinture.

La composition selon l'invention peut être une composition pour la teinture des cheveux. De telles compositions sont connues de l'homme du métier. On précise que les compositions pour la teinture des cheveux peuvent être constituées de plusieurs produits pour la teinture des cheveux, destinés à être mélangés par l'utilisateur. Dans la présente demande, sauf mention contraire ou précision particulière, le terme «composition pour la teinture de cheveux» couvre aussi bien une composition complète, ou un produit destiné à être mélangé à un autre par l'utilisateur. Dans la présente demande, le terme «teinture des cheveux», couvre toute modification de la couleur des cheveux, qu'il s'agisse d'une coloration proprement dite, d'une décoloration, ou d'une combinaison de décoloration et d'une coloration.

La composition pour la teinture des cheveux peut comprendre une base d'oxydation (précurseurs de colorants d'oxydation). Elle peut comprendre un agent d'oxydation. Elle peut comprendre un agent coupleur (modificateur de coloration). Elle peut comprendre un agent de coloration directe (colorants directs). La composition comprend un vecteur cosmétiquement acceptable. La composition peut comprendre également des adjuvants.

Selon un mode de réalisation, il s'agit d'une composition pour une teinture durable comprenant une base d'oxydation, un agent d'oxydation, et éventuellement un agent coupleur, de préférence sous forme de deux produits à associer, un produit comprenant la base d'oxydation et un produit comprenant l'agent d'oxydation.

Selon un mode de réalisation, il s'agit d'une composition pour une teinture temporaire ou durable comprenant un agent de coloration directe, et éventuellement un agent d'oxydation.

Selon un mode de réalisation, il s'agit d'une composition pour la décoloration ou l'éclaircissement des cheveux, comprenant un agent d'oxydation.

A titre d'agents de coloration directe, on peut citer les colorants nitrés benzéniques neutres, acides ou cationiques, les colorants directs azoïques neutres, acides ou cationiques, les colorants directs quinoniques et en particulier anthraquinoniques neutres acides ou cationiques, les colorants directs aziniques, les colorants directs méthiniques, les colorants directs tétraazapentaméthiniques, les colorants directs triarylméthaniques, les colorants directs indoaminiques et les colorants directs naturels.

A titre d'agents d'oxydation, on peut citer le peroxyde d'hydrogène, le peroxyde d'urée, les bromates de métaux alcalins, les persels tels que les perborates et les persulfates, les peracides et les enzymes, notamment les peroxydases,

les oxydoréductases à 2 électrons, et les oxygénases à 4 électrons.

A titre d'agents coupleurs, on peut citer les méthaphénylènediamines, les métaaminophénols, les métadiphénols, les coupleurs naphtaléniques et les coupleurs hétérocycliques.

A titre de vecteurs cosmétiquement acceptables, préférés dans les compositions de teinture, on peut citer l'eau et/ou ses mélanges avec des solvants, par exemple l'éthanol, l'isopropanol, les polyols et éthers de polyols comme le 2-butoxyéthanol, le propylène glycol, les alcools aromatiques comme l'alcool benzylique ou le phénoxyéthanol.

Les adjuvants peuvent être des tensioactifs anioniques, non-ioniques, cationiques ou zwitterioniques ou amphotères, des polymères anioniques, neutres ou cationiques, des agents épaississants minéraux ou organiques, des agents anti-oxydants, des agents de pénétration, des agents séquestrants, des parfums, des tampons, des agents dispersants, des agents de conditionnement, des agents filmogènes, des céramides, des agents conservateurs, des opacifiants. Bien entendu les ingrédients mentionnés plus haut peuvent être utilisés comme adjuvants dans les compositions pour la teinture.

Les compositions pour la teinture des cheveux comprenant le polyorganosiloxane de formule (1) peuvent ainsi :

- prévenir un éclaircissement de la coloration dans le temps (fading),
- favoriser une tenue de la coloration dans le temps,
- diminuer l'extraction de la couleur, et/ou
- réparer les cheveux vis à vis de l'oxydation.

Dans les produits de <u>protection solaire,</u> comprenant des filtres UV, par exemple de crèmes, laits, huiles, sprays solaires, le polyorganosiloxane de formule (I) peut avoir lui-même un effet de protection contre les effets des UV, sur la peau et/ou les cheveux. Il peut également avoir un effet d'amélioration de la protection d'autres agents, par exemple les filtres UV mentionnés ci-dessus, contre les effets des UV sur la peau et/ou sur les cheveux (synergie entre le polyorganosiloxane de formule (I) et d'autres agents). Les effets de protection contre les UV, peuvent également avoir un intérêt en ce qui concerne un maintien de l'aspect ou des performances de la composition dans le temps (moins de dégradations). Ainsi, le polyorganosiloxane de formule (I) peut éviter un jaunissement de la composition.

Les exemples présentés ci-dessous illustrent certains détails ou avantages de l'invention, sans caractère limitatif.

<u>EXEMPLES</u>

Dans les exemples ci-dessous l'ingrédient «Sil 1 » est une huile polyorganosiloxane de formule (I) dans laquelle:

- R est un groupe méthyle
- A est un méthyle
- Y est un groupe de formule (III)
- X est un groupe de formule $-(CH_2)_3-O-[OE]_{10}-[OP]_2$
- x=3
- n=4
- m =190.

Dans les exemples ci-dessous, la lettre C indique qu'il s'agit d'un exemple comparatif.

<u>Exemples 1-4</u>

Ces exemples illustrent des shampoings.

Les quantités indiquées sont des quantités en poids d'ingrédient.

<u>Procédure</u>

On dilue le Miracare CS (tensioactif amphotère) dans de l'eau contenant le Glydant sous agitation. On ajoute le silicone dans la phase aqueuse sous agitation. On ajuster le pH avec de l'acide citrique (solution à 30%).

| Exemple | 1 | 2C | 3C | 4C |
|---|---|---|---|---|
| Référence Echantillon: | 04 MMK 006 | 04 MMK 006A Témoin | 04 MMK 006B | 04 MMK 006C |
| Miracare CS, Rhodia | 37,40% | 37,40% | 37,40% | 37,40% |

(suite)

| Exemple | 1 | 2C | 3C | 4C |
|---|---|---|---|---|
| Sil 1 | 0,20% | | | |
| Mirasil ADM-E (émulsion à 29% de silicone) | | | 1 % | |
| Huile Mirasil DMCP 93, Rhodia (Silicone copolyol) | | | | 0,20% |
| Acide citrique (30% solution) | 0,70% | 0,70% | 0,70% | 0,70% |
| Glydant | 0,05% | 0,05% | 0,05% | 0,05% |
| Eau | 61,65% | 61,65% | 61,65% | 61,65% |
| PH | 5,20 | 5,19 | 5,15 | 5,21 |
| Viscosité(1) (cP) | 2400 | 15100 | 2520 | 5590 |
| Transmittance à $\lambda$ = 600 nm et jour = 0 | 99,11 | 99,15 | 99,24 | 99,22 |
| Absorbance à $\lambda$ 350 nm et jour = 0 | 0,2108 | 0,2119 | 0,2099 | 0,2109 |
| Absorbance à $\lambda$ 350 nm Après 21 jours à 45°C | 0,2334 | 0,2395 | 0,2750 | 0,2504 |
| Delta Absorbance (i.e. jaunissement à $\lambda$ 350 nm, après 21 jours à 45°C) | 0,0226 | 0,0276 | 0,0651 | 0,0395 |
| (1) Brookfield, (spindle 4, 10 tr $min^{-1}$ 25°C) | | | | |

Test de protection de la coloration contre l'irradiation solaire (Suntest)

Des mèches de 2 g de cheveux décolorés blond platine, de 15 cm de long sont pré-lavées avec une solution de lauryl sulfate de sodium diluée à 15% en matière active. Dans un grand bécher de 1 litre, on verse environ 900 ml de cette solution. On enfile les mèches sur un fil métallique et on les plonge dans la solution pendant 10 minutes. On les essorer légèrement puis on les positionne sur une grande plaque inclinée. On laisse couler l'eau pendant 45 minutes. L'eau est à 40°C avec un débit de 1,80 litres par minute. Après 45 minutes, les mèches sont démêlées avec un peigne jusqu'à ce qu'il n'y ait plus de noeuds.

On teint les mèches avec un gel colorant capillaire l'Oréal du commerce dont les références sont les suivantes: Colorant permanent Red Fiction n° 66 (coloration permanente groupe III). Les mèches sont rincées jusqu'à ce que l'eau soit claire en plaçant le ruban sur la paume de la main. On lave chaque mèche avec 0,5 g de shampoing Mixa Bébé pendant 1 minute et on rince chaque mèche pendant 1 minute. Après essorage entre deux doigts, les mèches sont séchées dans une salle climatique à 20°C et 50% RH pendant 12 heures. Chaque mèche est traitée avec un des 4 shampoings (exemples 1 à 4). On lave chaque mèche avec 0,5 g de shampooing pendant 1 minute et on rince chaque mèche pendant 1 minute. Après essorage entre deux doigts, les mèches sont séchées dans une salle climatique à 20°C et 50% RH pendant 12 heures.

Les couleurs des mèches sont évaluées dans le système L, a, b au moyen d'un colorimètre spectral Dr Lange utilisant le logiciel Luci 100 version 1.0 (Illuminant : C ; Géométrie de mesure : d/8° : Observateur standard : 10°). Pour chaque mèche on effectue la mesure sur une surface de (7*4) $cm^2$ que l'on a défini au préalable. Sur cette surface de mèche on effectue 6 mesures des coefficients L, a, b et on fait une moyenne des valeurs trouvées. Cette mesure est faite avant et après irradiation au Suntest.

Chaque mèche traitée est irradiée pendant 16 heures (ce qui correspond à 20 jours d'exposition naturelle) dans un Suntest Heraeus CPS+. Le Suntest permet de simuler, de manière accélérée, une exposition naturelle aux rayons UV et visible. Les conditions sont les suivantes : E = 500 $w/m^2$, la température = 20 +/- 5 °C, le taux d'humidité relative est de 25 +/- 5%, et la lampe utilisée est une lampe à Xénon.

On mesure dans le système L, a, b pour chaque mèche la couleur de la mèche obtenue après traitement par la composition (exemples 1 à 4) et la couleur de la mèche obtenue après le Suntest. L indique la clarté d'une couleur. Plus la valeur de L est élevée, plus la nuance est claire, a est la composante rouge-vert et b est la composante jaune-bleu. Les valeurs proches de zéro pour a ou b correspondent aux nuances grises.

La différence de couleur de la mèche traitée avant et après irradiation solaire dans le Suntest reflète la dégradation de la coloration. La dégradation est calculée en appliquant l'équation suivante:

$$\Delta E = \sqrt{(L - L_i)^2 + (a - a_i)^2 + (b - b_i)^2}$$

ΔE représente la différence de couleur entre deux mèches. $L_i$, $a_i$, et $b_i$, représentent respectivement la clarté, la nuance et la saturation avant irradiation. L, a, et b représentent respectivement la clarté, la nuance et la saturation après irradiation. Les résultats du test sont indiqués dans le tableau ci-dessous.

Résultats du Suntest

| | Couleur avant le Suntest | | | Couleur après le Suntest | | | Dégradation de la couleur ΔE |
|---|---|---|---|---|---|---|---|
| Traitement | $L_i$ | $a_i$ | $b_i$ | L | a | b | |
| Exemple 3C | 20,26 | 9,82 | 7,44 | 30,15 | 12,58 | 15,66 | 13,15 |
| Exemple 4C | 22,24 | 11,16 | 8,41 | 32,57 | 13,43 | 17,32 | 13,83 |
| Exemple 2C | 23,68 | 11,1 | 9,13 | 35,32 | 13,21 | 19,12 | 15,48 |
| Exemple 1 | 23,47 | 11,23 | 8,73 | 29,32 | 12,58 | 16,06 | 9,47 |

On constate que la dégradation de la couleur de la mèche traitée avec le shampooing selon l'invention (Exemple 1), après 16 heures d'irradiation solaire dans le Suntest, est réduite par réduite par rapport à celle obtenue avec les shampoings comparatifs.

Teste d'aide au démêlage des cheveux (conditionnement des cheveux)

A partir des bandeaux de cheveux décolorés blonde platine du fournisseur International Hair Importers Inc., on coupe à l'aide d'un gros cutter des mèches de largeur de 2,54cm. On prépare une solution de lauryl sulfate de sodium à 15% en matière active. Dans un grand bécher de 1litre, on verse environ 900 ml de cette solution. On enfile les mèches sur un fil métallique et on les plonge dans la solution pendant 10 minutes. On les essore légèrement puis on les positionne sur une grande plaque inclinée. On laisser couler de l'eau pendant 45 minutes. L'eau est à 40°C avec un débit de 1,80 litres par minute. Après 45 minutes, les mèches sont démêlée avec un peigne (côté écartement large) jusqu'à ce qu'il n'y ait plus de noeuds, sous l'eau du robinet. On suspend les mèches sur un rack, elles sont ainsi pré-traitées.

On détermine l'aptitude des shampoings à conditionner les cheveux à l'aide du dispositif Dia-Stron Miniature Tensile Tester MTT 170 et du logiciel Dia-Stron software « RHEOPC ». On suit un protocole adapté décrit dans les documents suivants: (1) Dia-Stron MTTWIN user manual, version 1.0 (June 1995) ; (2) M. L. Garcia et J. Diaz, « Combability measurements on human hair », J. Soc. Cosmet. Chem., 27, 379 (1976) et (3) Y. K. Kamath, et H. -D. Weigmann, « Measurement of combing forces », J. Soc. Cosmet. Chem., 37, 111 (1986).

Afin de préparer des groupes de mèches homogènes, la force de peignage est déterminée pour chaque mèche non-traitée mouillée. Chaque mèche non-traitée, mouillée est peignée 10 fois avec un peigne qui a 4 dents par centimètre. Elle est plongée dans un bécher d'eau dé-ionisée et l'excès d'eau est éliminé en la pinçant une fois entre l'index et le majeur. Cette procédure induit de légers enchevêtrements entre les fibres de cheveux. Chaque mèche est peignée 6 fois en utilisant le mobile de peignage du Dia-Stron MTT 170 et la moyenne de la force de peignage mouillé, non-traité ($F_{MNT}$) est calculée. Les groupes de mèches sont constitués de manière homogène à l'aide d'une analyse statistique.

Le traitement des mèches se déroule de la manière suivante: Les mèches du même groupe à traiter sont immergées dans un grand bécher d'eau du robinet pendant une minute. L'excès d'eau est éliminé en pinçant la mèche une fois entre l'index et le majeur. 0.2ml de shampooing pour 2g de cheveux sont déposés à l'aide d'une seringue. Chaque mèche est massée 30 fois pendant 1 minute. Les mèches traitées sont enfilée sur un fil métallique et positionnées sur une grande plaque inclinée. On laisse couler de l'eau pendant 1 minute. L'eau est à 40°C avec un débit de 1,80 litres par minute. Les mèches traitées sont prêtes pour être évaluées en test de peignage mouillé traité « Treated Wet Combing ». Chaque mèche traitée est peignée 6 fois utilisant le mobile du peignage du Dia-Stron MTT 170 et la moyenne de la force de peignage mouillé, traitée ($F_{MT}$) est calculée pour les groupes de mèches.

La différence de la moyenne de la force de peignage du groupe de mèches mouillées avant et après traitement reflète le conditionnement des cheveux (facilité à démêler les cheveux mouillés). Le conditionnement est calculé en appliquant l'équation suivante:

$$\Delta F = F_{MT} - F_{MNT}$$

ΔF représente la différence de la moyenne de la force de peignage du groupe de mèches mouillées avant et après traitement. $F_{MT}$ représente la moyenne de la force de peignage du groupe des mèches mouillées, traitées. $F_{MNT}$ représente la moyenne de la force de peignage du groupe des mèches mouillées, non-traitées. Plus la valeur de ΔF est négative, plus le traitement est efficace. Le résultat du test est indiqué dans le tableau ci-dessous.

Résultat du teste d'aide au démêlage des cheveux (conditionnement des cheveux)

| **Traitement** | **$F_{MNT}$ moyenne de la force de peignage du groupe des mèches mouillées, non-traitées** | **$F_{MT}$ moyenne de la force de peignage du groupe des mèches mouillées, traitées** | **ΔF Conditionnement des cheveux** |
|---|---|---|---|
| Exemple 1 | 0,280 J | 0,260 J | -0,020 J |

On constate un conditionnement des cheveux mouillés.

Exemples 5-9

Ces exemples illustrent des sprays de brillance («shine spray»).

On réalise les compositions suivantes (les quantités sont indiquées en % en poids d'ingrédient):

On mélange le Mirasil PTM et le silicone dans le Mirasil CM5 sous agitation. On ajoute l'ethanol et on agite la solution.

| | Exemple | 5C | 6C | 7C | 8 | 9C |
|---|---|---|---|---|---|---|
| | Référence Echantillon | 04 MMK 003 ADME | 04 MMK 003 ADM | 04 MMK 003 DMCP 93 | 04 MMK 003 HALS copo | Blank |
| Quantités | Ingrédients | | | | | |
| 5,00% | Mirasil PTM, Rhodia | | | | | |
| 39,00% | Mirasil CM5, Rhodia | | | | | |
| 1,00% | Silicone | Mirasil ADME, Rhodia | Mirasil ADM, Rhodia | Mirasil DMCP 93, Rhodia | Sil 1 | Rien |
| 55,00% | Ethanol | | | | | |
| | | | | | | |
| aspect | | 2 phases (toutes deux claires) | 2 phases (touts deux claires) | 1 phase (turbide) | 2 phases (toutes deux claires) | 1 phase (claire) |

On mélange le Mirasil PTM et le silicone dans le Mirasil CM5 sous agitation. On ajoute l'éthanol et on agite la solution.

Exemples 10-11

Ces exemples illustrent des crèmes solaires.

Les quantités sont indiquées % en poids d'ingrédient.

| | Exemple | 10C | 11 |
|---|---|---|---|
| | Référence Echantillon | 04 MMK 008 | 04 MMK 009 |
| | Ingrédients | | |
| Phase 1 | Brij 72, Uniqema | 2 % | 2 % |
| Phase 1 | Brij 721 P, Uniqema | 4 % | 4 % |
| Phase 1 | Elefac I 205, National Starch | 4 % | 4 % |
| Phase 1 | Cetiol CC, Cognis | 4 % | 4 % |
| Phase 1 | Eusolex 2292, Merck | 7,5 % | 7,5 % |
| Phase 1 | Parsol 1789, Givaudan Roure | 1,5 % | 1,5 % |
| Phase 1 | Mirasil Wax B, Rhodia | 4 % | 4 % |
| Phase 1 | Mirasil CM5, Rhodia | 4 % | 4 % |
| Phase 1 | Carnation, Crompton | 4 % | 0 % |
| Phase 1 | Sil 1 | 0 % | 4 % |
| Phase 1 | Vitamine E Acetate | 0,3 % | 0,3 % |
| Phase 2 | Dermacryl 79, National Starch | 2 % | 2 % |
| Phase 2 | TEA 99% | 0,67 % | 0,67 % |
| Phase 2 | Deionized water | 10 % | 10 % |
| Phase 3 | Sepigel 501, Seppic | 1 % | 1 % |
| Phase 3 | Glycerine | 1 % | 1 % |
| Phase 3 | Propylene Glycol, Lyondell | 1 % | 1 % |
| Phase 3 | Allantoin | 0,2% | 0,2 % |
| Phase 3 | Germaben II-E, ISP | 0,2 % | 0,2 % |
| Phase 3 | Eau deionisée | 35,33% | 35,33 % |
| Phase 4 | Mirasun TIW 60, Rhodia | 13,3% | 13,3 % |
| Phase 5 | Acide Citrique (50% solution) | q.s. | q.s. |
| | pH | 6,80 | 6,91 |
| | Viscosité (1)cP | 5950 | 8300 |
| (1) Brookfield, (spindle 6, 20 tr $min.^{-1}$, 25°C) | | | |

Procédure

1) Préparer la phase 2: disperser Dermacryl dans eau contenant TEA sous agitation
2) Préparer la phase 3: disperser Sepigel 501 dans eau sous agitation et homogénéiser, puis ajouter conservateur, Allantoin, glycérine et propylène glycol.
3) Ajouter la phase 2 dans la phase 3.
4) Préparer la phase 1: Mélanger et chauffer à 70°C les ingrédients et homogénéiser.
5) Ajouter la phase 1 dans les phase 2 et 3, à même température, et agiter avec Turrax environ 2 minutes. Ajouter Mirasun TIW 60 sous agitation. Refroidir l'émulsion obtenue à 30°C sous agitation et ajuster le pH à 6,8 avec l'acide citrique.

Test de la crème solaire

La crème solaire à analyser est déposée à raison de 55 à 60 mg sur une bande scotch TRANSPORE 3M. Elle est ensuite étalée de façon à ce que le film de crème soit homogène sur toute la longueur de la bande scotch TRANSPORE 3M. La mesure du facteur de protection solaire (SPF) se fait dans une salle climatisée à humidité contrôlée. L'appareil utilisé, Optometrics SPF 290, est calibré et le blanc réalisé sur une bande scotch TRANSPORE 3M. Deux mesures indépendantes sont effectuées par crème solaire (chacune est la moyenne de neuf mesures par bande de TRANSPORE 3M). Les résultats sont présentés dans le tableau ci-dessous. Ils montrent une augmentation de l'indice de protection (I.P.) moyen pour la crème solaire selon l'invention (Exemple 11).

| Echantillon | Moyenne I.P. In Vitro | Ecart type | moyenne UVA | ratio UVA/UVB |
|---|---|---|---|---|
| Exemple 10C | 21,7 | 3,0 | 13,8 +/- 2,6 | 0,70 +/- 0,04 |
| Exemple 10C | 20,9 | 1,8 | 13,0 +/- 1,0 | 0,70 +/- 0,04 |
| Exemple 11 | 40,4 | 1,6 | 18,6 +/- 1,1 | 0,66 +/- 0,02 |
| Exemple 11 | 43,3 | 5,3 | 21,8 +/- 2,6 | 0,68 +/- 0,01 |

Exemples 12-15

Ces exemples illustrent des revêtements de cuticule. («cuticle coat»). Les quantités sont indiquées en % en poids d'ingrédient.

| | Exemple | 12C | 13C | 14C | 15 |
|---|---|---|---|---|---|
| | Référence Echantillon | 04 MMK 019 Témoin | 04 MMK 019 ADM | 04 MMK 019 DMCP 93 | 04 MMK 019 HALS copolyol |
| quantités | Ingrédients | | | | |
| 2,00% | Mirasil PTM, Rhodia | | | | |
| 16,00% | Mirasil CM5. Rhodia | | | | |
| 2,00% | Silicone | Rien | Mirasil ADM, Rhodia | Mirasil DMCP 93, Rhodia | Sil 1 |
| 80,00% | Mirasil C-DML, Rhodia | | | | |
| | | | | | |
| aspect | | 1 phase (claire, visqueux) | 1 phase (claire, visqueux) | 1 phase (claire, visqueux) | 1 phase (claire, visqueux) |

On mélange le Mirasil PTM et le silicone dans le Mirasil CM5 sous agitation. On ajouter le Mirasil C-DML et on malaxe afin de mélanger les huiles silicones.

## Revendications

1. Composition cosmétique comprenant un vecteur cosmétiquement acceptable et un polyorganosiloxane, **caractérisée en ce que** le polyorganosiloxane présente la formule générale (I) suivante:

$$[R_aX_bY_cSiO_{(4-a-b-c)/2}]_N \qquad (I)$$

dans laquelle:

- $R_aX_bY_cSiO_{(4-a-b-c)/2}$, identiques ou différents, représentent des motifs, linéaires ou branchés, compris dans

le polyorganosiloxane,
- N représente le nombre d'atomes de silicium compris dans le polyorganosiloxane, supérieur ou égal à 3,
- a, b, et c, identiques ou différents, sont des nombres égaux à 0, 1, 2 ou 3,
- a+b+c, identique ou différent, est égal à 0, 1, 2 ou 3,
- R, identique ou différent, représente un groupe alkyle en $C_1$-$C_{18}$, un groupe aryle en $C_6$-$C_{12}$, un groupe aralkyle $C_6$-$C_{12}$, un groupe alkylaryl en $C_6$-$C_{12}$, un groupe de formule
- $[CH_2]_3$-$NH[CH_2]_2$-$NH_2$, un groupe de formule -$[CH_2]_3$-$NH_2$, un groupe hydroxyle, un groupe alcoxy en $C_1$-$C_{18}$, un groupe hydroxyalkyl $C_1$-$C_{18}$, ou un groupe hydroxyalkyletheralkyl en $C_1$-$C_{18}$, lesdits groupes étant éventuellement substitués,
- X, identique ou différent, représente un groupe polyalcoxylé porté par un atome de silicium,
- Y, identique ou différent, représente un groupe porté par un atome de silicium, de formule -$R^4$-U-Hals, dans laquelle:

  - $R^4$ est un groupe de liaison divalent hydrocarboné, de préférence un groupe alkyle,
  - U est une liaison covalente ou un groupe rotule divalent comprenant un hétéroatome,
  - Hals est un groupe fonctionnel comprenant une amine stériquement encombrée,

- au moins un atome de silicium du polyorganosiloxane porte un groupe X, et
- au moins un atome de silicium du polyorganosiloxane porte un groupe Y.

**2.** Composition selon la revendication 1, **caractérisé en ce que** les groupes X et Y sont portés par des atomes de silicium différents.

**3.** Composition selon l'une des revendications précédentes, **caractérisée en ce que** le groupe X présente la formule $L^X$-$Z^x$-Palc, dans laquelle:

- $L^X$ est un groupe de liaison divalent, de préférence un groupe alkyle,
- est une liaison covalente ou un groupe rotule divalent comprenant un hétéroatome,
- Palc est un groupe de formule $[OE]_s$-$[OP]_t$-X', dans laquelle

  - OE est un groupe de formule -$CH_2$-$CH_2$-O-
  - OP est un groupe de formule -$CH_2$-$CHCH_3$-O- ou -$CHCH_3$-$CH_2$-O-,
  - X' est un atome d'hydrogène ou un groupe de hydrocarboné,
  - s est un nombre moyen supérieur à 1, et
  - t est un nombre moyen supérieur ou égal à 0,

**4.** Composition selon l'une des revendications précédentes, **caractérisée en ce que** X est un groupe de formule -$CH_2$-$CH_2$-$CH_2$-O-$[OE]_q$-$[OP]_r$-X', dans laquelle:

- $q \geq 5$,
- $r \geq 0$, et
- X' est un atome d'hydrogène.

**5.** Composition selon l'une des revendications précédentes **caractérisée en ce que** le groupe Y présente la formule (II) suivante:

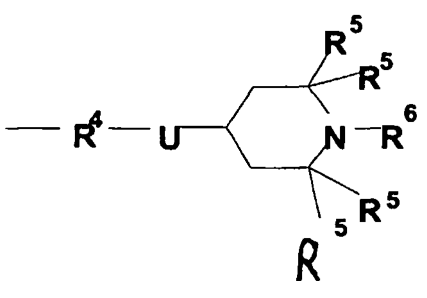

(II)

dans laquelle:

- $R^4$, identique ou différent, est un divalent hydrocarboné divalent choisi parmi:

  - les groupes alkylènes linéaires ou ramifiées, ayant 2 à 18 atomes de carbone;
  - les groupes alkylène-carbonyle dont la partie alkylène, linéaire ou ramifiée, comprend 2 à 20 atomes de carbone;
  - les groupes alkylène-cyclohexylène dont la partie alkylène linéaire ou ramifiée, comprend 2 à 12 atomes de carbone et la partie cyclo-hexylène comporte un groupe -OH et éventuellement 1 ou 2 groupes alkyles ayant 1 à 4 atomes de carbone;
  - les groupes de formule $-R^7-O-R^7$ dans laquelle les groupes $R^7$, identiques ou différents, représentent des groupes alkylènes ayant 1 à 12 atomes de carbone;
  - les groupes de formule $-R^7-O-R^7$ dans laquelle les groupes $R^7$ ont les significations indiquées précédemment et l'un d'entre eux ou les deux sont substitués par un ou deux groupe(s) -OH;
  - les groupes de formule $-R^7-COO-R^7$ dans laquelle les groupes $R^7$ ont les significations indiquées précédemment; et
  - les groupes de formule $-R^8-O-R^9-O-CO-R^8$ dans laquelle les groupes $R^8$ et $R^9$, identiques ou différents, représentent des groupes alkylènes ayant 2 à 12 atomes de carbone et le groupe $R^9$ est éventuellement substitué par un groupe hydroxyle;

- U représente -O- ou $-NR^{10}-$, $R^{10}$ étant un atome d'hydrogène, un groupe alkyle linéaire ou ramifié comprenant 1 à 6 atomes de carbone;
- $R^5$, identique ou différent, est un groupe alkyle alinéaire ou ramifié ayant 1 à 3 atomes de carbone ou un groupe phényle; et
- $R^6$ représente un atome d'hydrogène, un groupe $R^5$ ou un radical libre O°.

6. Composition selon l'une des revendications précédentes, **caractérisée en ce que** le groupe Y est un groupe de formule (III) suivante:

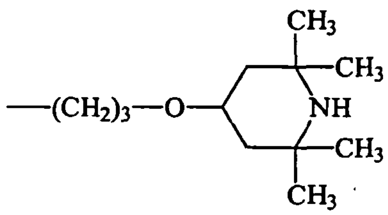

(III)

7. Composition selon l'une des revendications précédentes, **caractérisée en ce que** le groupe R, identique ou différent, est un groupe méthyle, éthyle, isopropyle, tertiobutyle, n-hexyle, octyle, trifluoropropyle, ou phényle.

8. Composition selon l'une des revendications précédentes, **caractérisée en ce que** R est un groupe méthyle.

9. Composition selon l'une des revendications précédentes, **caractérisée en ce que** le polyorganosiloxane de formule (I) est un polyorganosiloxane linéaire.

10. Composition selon l'une des revendications précédentes, **caractérisée en ce que** le polyorganosiloxane de formule (I) présente la formule (IV) suivante:

$$A-SiR_2-O-[SiRXO]_x-[SiRYO]_n-[SiR_2O]_m-O-SiR_2-A \qquad (IV)$$

dans laquelle:

- R, identique ou différent, est un groupe tel que défini dans l'une des revendications précédentes,
- X, identique ou différent, est un groupe tel que défini dans l'une des revendications précédentes,
- Y, identique ou différent, est un groupe tel que défini dans l'une des revendications précédentes,
- A, identique ou différent, est un groupe R, Y, ou X,
- x est un nombre moyen supérieur à 0,
- n est un nombre moyen supérieur à 0,
- m est un nombre moyen supérieur à 0, et

- x+m+n+2 = N.

**11.** Composition selon la revendication 10, **caractérisée en ce que**:

- A est un groupe R, et
- R est un groupe méthyle.

**12.** Composition selon l'une des revendications 10 ou 11, **caractérisée en ce que**:

- $m \geq 10$, de préférence $m \geq 100$
- $x \geq 1$, et
- $n \geq 3$.

**13.** Composition selon l'une des revendications 10 à 12, **caractérisée en ce que** le polyorganosiloxane de formule (I) comprend entre 0,1 et 0,5 % en poids d'atomes d'azote.

**14.** Composition selon l'une des revendications 10 à 13, **caractérisée en ce que**:

- $m \geq 50$
- $0{,}001 \leq n/(m+n+x) \leq 0{,}5$, de préférence $0{,}005 \leq n/(m+n+x) \leq 0{,}1$.

**15.** Composition selon l'une des revendications 10 à 14, **caractérisée en ce que**:

- $m \geq 50$
- $0{,}001 \leq x/(m+n+x) \leq 0{,}5$, de préférence $0{,}01 \leq x/(m+n+x) \leq 0{,}2$.

**16.** Composition selon l'une des revendications précédentes, **caractérisée en ce que** le polyorganosiloxane de formule (I) présente une viscosité comprise entre 1000 et 1000000 mPa.s, de préférence entre 5000 et 500000 mPa.s, de préférence entre 10000 et 100000 mPa.s.

**17.** Composition selon l'une des revendications précédentes, **caractérisée en ce que** le polyorganosiloxane est présent dans la composition sous forme d'une émulsion de gouttelettes comprenant le polyorganosiloxane de formule (I), dispersées dans un vecteur cosmétiquement acceptable aqueux.

**18.** Composition selon l'une des revendications précédentes, **caractérisée en ce que**:

- l'émulsion est une émulsion dont la taille moyenne des gouttelettes est supérieure ou égale à 0,15 μm, obtenue à l'aide d'un agent d'émulsification du polyorganosiloxane de formule (I), de préférence en proportion inférieure à 10%, en poids par rapport à la quantité de polyorganosiloxane de formule (I), ou
- l'émulsion est une microémulsion dont la taille moyenne des gouttelettes est inférieure à 0,15 μm, obtenue à l'aide d'un agent d'émulsification du polyorganosiloxane de formule (I), de préférence en proportion supérieure à 10% en poids par rapport à la quantité de polyorganosiloxane de formule (I).

**19.** Composition selon la revendication précédente, **caractérisée en ce qu'**elle comprend en plus du polyorganosiloxane de formule (I):

- au moins un tensioactif anionique et/ou amphotère, seul ou en mélange,
- éventuellement, au moins un agent de stabilisation et/ou de conditionnement et/ou d'aide au conditionnement, ou un mélange de tels agents,
- éventuellement un autre polyorganosiloxane,
- éventuellement un filtre UV.

**20.** Composition selon la revendication 19, **caractérisée en ce que** l'agent de stabilisation et/ou de conditionnement et/ou d'aide au conditionnement est choisi parmi:

- les polymères cationiques dérivés de polysaccharides, par exemple les dérivés cationiques de celluloses, les dérivés cationiques d'amidons, les dérivés cationiques de guars, les dérivés cationique de caroube,
- les polymères cationiques synthétiques,

- les agents stabilisants, de préférence choisis parmi les polyacrylates réticulés et solides insolubles formant un réseau dans la composition, par exemple l'éthylène glycol distéarate (EGDS),
- les mélanges ou combinaisons de ces agents.

**21.** Composition selon l'une des revendications précédentes, **caractérisée en ce qu'**il s'agit d'une composition cométique pour le soin de la peau et/ou des cheveux, de préférence pour de nettoyage et/ou le traitement et la peau et/ou des cheveux, ladite composition étant sous forme d'un fluide.

**22.** Composition selon l'une des revendications précédentes, **caractérisée en ce que en ce qu'**il s'agit d'un gel douche, d'un shampoing, d'un après-shampoing, destiné à être rincé ou non.

**23.** Composition selon l'une des revendications précédentes, **caractérisée en ce qu'**il s'agit d'une produit de protection solaire.

## Claims

**1.** A cosmetic composition comprising a cosmetically acceptable carrier and a polyorganosiloxane, **characterized in that** the polyorganosiloxane has the following general formula (I) :

$$[R_aX_bY_cSiO_{(4-a-b-c)/2}]_N \qquad (I)$$

where:

- $R_aX_bY_cSiO_{(4-a-b-c)/2}$, which may be identical or different, represent linear or branched units in the polyorganosiloxane,
- N represents the number of silicon atoms in the polyorganosiloxane, greater than or equal to 3,
- a, b and c, which may be identical or different, are numbers equal to 0, 1, 2 or 3,
- a + b + c, which may be identical or different, is equal to 0, 1, 2 or 3,
- R, which may be identical or different, represents a $C_1$-$C_{18}$ alkyl group, a $C_6$-$C_{12}$ aryl group, a $C_6$-$C_{12}$ aralkyl group, a $C_6$-$C_{12}$ alkaryl group, a group of formula
- $[CH_2]_3$-$NH[CH_2]_2$-$NH_2$, a group of formula -$[CH_2]_3$-$NH_2$, a hydroxyl group, a $C_1$-$C_{18}$ alkoxy group, a $C_1$-$C_{18}$ hydroxyalkyl group, or a $C_1$-$C_{18}$ hydroxyalkyletheralkyl group, said groups being optionally substituted,
- X, which may be identical or different, represents a polyalkoxy group carried by a silicon atom,
- Y, which may be identical or different, represents a group carried by a silicon atom, of formula -$R^4$-U-Hals, in which:

  - $R^4$ is a divalent hydrocarbon binding group, preferably an alkyl group,
  - U is a covalent bond or a divalent swivel group comprising a heteroatom,
  - Hals is a functional group comprising a sterically hindered amine,

- at least one silicon atom of the polyorganosiloxane bears a group X, and
- at least one silicon atom of the polyorganosiloxane bears a group Y.

**2.** The composition as claimed in claim 1, **characterized in that** the groups X and Y are carried by different silicon atoms.

**3.** The composition as claimed in either of the preceding claims, **characterized in that** the group X has the formula L"Z"-Palk, in which:

- $L^x$ is a divalent bonding group, preferably an alkyl group,
- $Z^x$ is a covalent bond or a divalent connecting group comprising a heteroatom,
- Palk is a group of formula $[OE]_s$-$[OP]_t$-X', in which

  - OE is a group of formula -$CH_2$-$CH_2$-O-,
  - OP is a group of formula -$CH_2$-$CHCH_3$-O- or
  - $CHCH_3$-$CH_2$-O-,
  - X' is a hydrogen atom or a hydrocarbon-based group,
  - s is an average number greater than 1, and

- t is an average number greater than or equal to 0.

**4.** The composition as claimed in one of the preceding claims, **characterized in that** X is a group of formula $-CH_2-CH_2-CH_2-O-[OE]_q-[OP]_r-X'$, in which:

- $q \geq 5$,
- $r \geq 0$, and
- x' is a hydrogen atom.

**5.** The composition as claimed in one of the preceding claims, **characterized in that** group Y has the following formula (II):

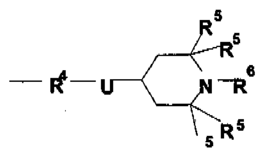

(II)

in which:

- $R^4$, which may be identical or different, is a divalent hydrocarbon divalent selected from:
  - linear or branched alkylene groups having 2 to 18 carbon atoms;
  - alkylene-carbonyl groups, the linear or branched alkylene portion of which has 2 to 20 carbon atoms;
  - alkylene-cyclohexylene groups, the linear or branched alkylene portion of which has 2 to 12 carbon atoms and the cyclohexylene portion has an
  - OH group and optionally 1 or 2 alkyl groups having 1 to 4 carbon atoms;
  - groups of formula $-R^7-O-R^7$ in which the $R^7$ groups, which may be identical or different, represent alkylene groups having 1 to 12 carbon atoms;
  - groups of formula $R^7-O-R^7$ in which the $R^7$ groups have the meanings stated previously and one of them or both of them are substituted with one or two -OH groups;
  - groups of formula $-R^7-COO-R^7$ in which the $R^7$ groups have the meanings stated previously; and
  - groups of formula $-R^8-O-R^9-O-CO-R^8$ in which the $R^8$ and $R^9$ groups, which may be identical or different, represent alkylene groups having 2 to 12 carbon atoms and the $R^9$ group is optionally substituted with a hydroxyl group;
- U represents -O- or $-NR^{10}-$, $R^{10}$ being a hydrogen atom, a linear or branched alkyl group comprising 1 to 6 carbon atoms;
- $R^5$, which may be identical or different, is a linear or branched alkyl group having 1 to 3 carbon atoms or a phenyl group; and
- $R^6$ represents a hydrogen atom, an $R^5$ group or a free radical O°.

**6.** The composition as claimed in one of the preceding claims, **characterized in that** group Y is a group of the following formula (III):

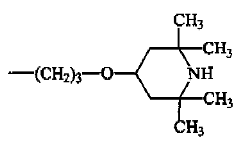

(III)

**7.** The composition as claimed in one of the preceding claims, **characterized in that** group R, which may be identical or different, is a methyl, ethyl, isopropyl, tert-butyl, n-hexyl, octyl, trifluoropropyl, or phenyl group.

**8.** The composition as claimed in one of the preceding claims, **characterized in that** R is a methyl group.

**9.** The composition as claimed in one of the preceding claims, **characterized in that** the polyorganosiloxane of formula (I) is a linear polyorganosiloxane.

**10.** The composition as claimed in one of the preceding claims, **characterized in that** the polyorganosiloxane of formula (I) has the following formula (IV):

$$A\text{-}SiR_2\text{-}O\text{-}[SiRXO]_x\text{-}[SiRYO]_n\text{-}[SiR_2O]_m\text{-}O\text{-}SiR_2\text{-}A \qquad (IV)$$

in which:

- R, which may be identical or different, is a group as defined in one of the preceding claims,
- X, which may be identical or different, is a group as defined in one of the preceding claims,
- Y, which may be identical or different, is a group as defined in one of the preceding claims,
- A, which may be identical or different, is a group R, Y, or X,
- x is an average number greater than 0,
- n is an average number greater than 0,
- m is an average number greater than 0, and
- x+m+n+2 = N.

**11.** The composition as claimed in claim 10, **characterized in that**:

- A is a group R, and
- R is a methyl group.

**12.** The composition as claimed in one of claims 10 or 11, **characterized in that**:

- $m \geq 10$, preferably $m \geq 100$,
- $x \geq 1$, and
- $n \geq 3$.

**13.** The composition as claimed in one of claims 10 to 12, **characterized in that** the polyorganosiloxane of formula (I) comprises between 0.1 and 0.5 wt.% of nitrogen atoms.

**14.** The composition as claimed in one of claims 10 to 13, **characterized in that**:

- $m \geq 50$
- $0.0001 \leq n/(m+n+x) \leq 0.5$, preferably $0.005 \leq n/(m+n+x) \leq 0.1$.

**15.** The composition as claimed in one of claims 10 to 14, **characterized in that**:

- $m \geq 50$
- $0.001 \leq x(m+n+x) \leq 0.5$, preferably $0.01 \leq x/(m+n+x) \leq 0.2$.

**16.** The composition as claimed in one of the preceding claims, **characterized in that** the polyorganosiloxane of formula (I) has a viscosity between 1000 and 1000000 mPa.s, preferably between 5000 and 500000 mPa.s, preferably between 10000 and 100000 mPa.s.

**17.** The composition as claimed in one of the preceding claims, **characterized in that** the polyorganosiloxane is present in the composition in the form of an emulsion of droplets comprising the polyorganosiloxane of formula (I), dispersed in a cosmetically acceptable aqueous carrier.

**18.** The composition as claimed in one of the preceding claims, **characterized in that**:

- the emulsion is an emulsion with average droplet size greater than or equal to 0.15 μm, obtained using an emulsifier of the polyorganosiloxane of formula (I), preferably in a proportion less than 10 wt.% relative to the amount of polyorganosiloxane of formula (I), or
- the emulsion is a microemulsion with average droplet size less than 0.15 μm, obtained using an emulsifier of the polyorganosiloxane of formula (I), preferably in a proportion greater than 10 wt.% relative to the amount of

polyorganosiloxane of formula (I).

**19.** The composition as claimed in the preceding claim, **characterized in that** it comprises, in addition to the polyorganosiloxane of formula (I):

- at least one anionic and/or amphoteric surfactant, alone or mixed,
- optionally, at least one stabilizer and/or conditioner and/or conditioning aid, or a mixture of such agents,
- optionally another polyorganosiloxane,
- optionally a UV filter.

**20.** The composition as claimed in claim 19, **characterized in that** the stabilizer and/or conditioner and/or conditioning aid is selected from:

- cationic polymers derived from polysaccharides, for example cationic derivatives of celluloses, cationic derivatives of starches, cationic derivatives of guars, cationic derivatives of carob,
- synthetic cationic polymers,
- stabilizers, preferably selected from the crosslinked polyacrylates and insoluble solids forming a network in the composition, for example ethylene glycol distearate (EGDS),
- mixtures or combinations of these agents.

**21.** The composition as claimed in one of the preceding claims, **characterized in that** it is a cosmetic composition for care of the skin and/or of the hair, preferably for cleaning and/or treatment of the skin and/or of the hair, said composition being in the form of a fluid.

**22.** The composition as claimed in one of the preceding claims, **characterized in that** it is a shower gel, a shampoo, an after-shampoo, intended to be rinsed or not.

**23.** The composition as claimed in one of the preceding claims, **characterized in that** it is a sun protection product.

## Patentansprüche

**1.** Kosmetische Zusammensetzung, welche einen kosmetisch verträglichen Träger und ein Polyorganosiloxan umfasst, **dadurch gekennzeichnet, dass** das Polyorganosiloxan die folgende allgemeine Formel (I) aufweist:

$$[R_aX_bY_cSiO_{(4-a-b-c)/2}]_N \qquad (I),$$

in welcher:

- $R_aX_bY_cSiO_{(4-a-b-c)/2}$, die gleich oder verschieden sind, für lineare oder verzweigte Motive, die in dem Polyorganosiloxan enthalten sind, stehen,
- N für die Anzahl von Siliciumatomen, die in dem Polyorganosiloxan enthalten sind, welche höher oder gleich 3 Ist, steht,
- a, b und c, die gleich oder verschieden sind, Zahlen, die gleich 0, 1, 2 oder 3 sind, sind,
- a+b+c, das gleich oder verschieden ist, gleich 0, 1, 2 oder 3 ist,
- R, das gleich oder verschieden ist, für eine $C_1$-$C_{18}$-Alkylgruppe, eine $C_6$-$C_{12}$-Arylgruppe, eine $C_6$-$C_{12}$-Arylalkylgruppe, eine $C_6$-$C_{12}$-Alkylarylgruppe, eine Gruppe der Formel $-[CH_2]_3-NH[CH_2]_2-NH_2$, eine Gruppe der Formel $-[CH_2]_3-NH_2$, eine Hydroxylgruppe, eine $C_1$-$C_{18}$-Alkoxygruppe, eine $C_1$-$C_{18}$-Hydroxyalkylgruppe oder eine $C_1$-$C_{18}$-Hydroxyalkyletheralkylgruppe, wobei diese Gruppen gegebenenfalls substituiert sind, steht,
- X, das gleich oder verschieden ist, für eine polyalkoxylierte Gruppe steht, die von einem Siliciumatom getragen wird,
- Y, das gleich oder verschieden ist, für eine Gruppe steht, die von einem Siliciumatom getragen wird, mit der Formel $-R^4$-U-Hals, In welcher:
    - $R^4$ eine zweiwertige, Kohlenwasserstoff-Verbindungsgruppe, vorzugsweise eine Alkylgruppe, Ist,
    - U eine kovalente Bindung oder eine zweiwertige Gelenkgruppe, welche ein Heteroatom umfasst, ist,
    - Hals eine funktionelle Gruppe ist, weiche ein sterisch gehindertes Amin umfasst,

- mindestens ein Siliciumatom des Polyorganosiloxans eine Gruppe X trägt und
- mindestens ein Siliciumatom des Polyorganosiloxans eine Gruppe Y trägt.

**2.** Zusammensetzung nach Anspruch 1, **dadurch gekennzeichnet, dass** die Gruppen X und Y von unterschiedlichen Siliciumatomen getragen werden.

**3.** Zusammensetzung nach einem der vorangegangenen Ansprüche, **dadurch gekennzeichnet, dass** die Gruppe X die Formel $L^x$-$Z^x$-Palc aufweist, in welcher:

- $L^x$ eine zweiwertige Verbindungsgruppe, vorzugsweise eine Alkylgruppe, ist,
- $Z^x$ eine kovalente Bindung oder eine zweiwertige Gelenkgruppe, welche ein Heteroatom umfasst, Ist,
- Palc eine Gruppe der Formel $[OE]_s$-$[OP]_t$-X' ist, in welcher

  - OE eine Gruppe der Formel $-CH_2-CH_2-O-$ Ist,
  - OP eine Gruppe der Formel $-CH_2-CHCH_3-O-$ oder $-CHCH_3-CH_2-O-$ ist,
  - X' ein Wasserstoffatom oder eine Kohlenwasserstoffgruppe Ist,
  - s eine mittlere Zahl über 1 ist und
  - t eine mittlere Zahl über oder gleich 0 ist.

**4.** Zusammensetzung nach einem der vorangegangenen Ansprüche, **dadurch gekennzeichnet, dass** X eine Gruppe der Formel $-CH_2-CH_2-CH_2-O-[OE]_q-[OP]_r-X'$ ist, in welcher:

- $q \geq 5$,
- $r \geq 0$ und
- X' ein Wasserstoffatom ist.

**5.** Zusammensetzung nach einem der vorangegangenen Ansprüche, **dadurch gekennzeichnet, dass** die Gruppe Y die folgende Formel (II) aufweist:

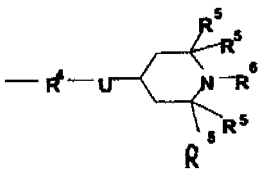

(II) ;

in welcher:

- $R^4$, das gleich oder verschieden ist, eine zweiwertige, KohlenwasserstoffGruppe ist, die ausgewählt ist unter:

  - den linearen oder verzweigten Alkylengruppen mit 2 bis 18 Kohlenstoffatomen;
  - den Alkylencarbonylgruppen, deren Alkylenanteil, welcher linear oder verzweigt Ist, 2 bis 20 Kohlenstoffatome umfasst;
  - den Alkylencyclohexylengruppen, deren Alkylenanteil, der linear oder verzweigt ist, 2 bis 12 Kohlenstoffatome umfasst und deren Cyclohexylenanteil eine Gruppe -OH und gegebenenfalls 1 oder 2 Alkylgruppen mit 1 bis 4 Kohlenstoffatomen umfasst;
  - den Gruppen der Formel $-R^7-O-R^7$, in welcher die Gruppen $R^7$, die gleich oder verschieden sind, für Alkylengruppen mit 1 bis 12 Kohlenstoffatomen stehen;
  - den Gruppen der Formel $-R^7-O-R^7$, in welcher die Gruppen $R^7$ die zuvor angegebenen Bedeutungen haben und eine von diesen oder beide durch eine oder zwei Gruppe(n) -OH substituiert sind;
  - den Gruppen der Formel $-R^7-COO-R^7$, in welcher die Gruppen $R^7$ die zuvor angegebenen Bedeutungen haben; und
  - den Gruppen der Formel $-R^8-O-R^9-O-CO-R^8$, in welcher die Gruppen $R^8$ und $R^9$, die gleich oder verschieden sind, für Alkylengruppen mit 2 bis 12 Kohlenstoffatomen stehen und die Gruppe $R^9$ gegebenenfalls durch eine Hydroxylgruppe substituiert ist;

- U für -O- oder $-NR^{10}-$ steht, wobei $R^{10}$ ein Wasserstoffatom, eine lineare oder verzweigte Alkylgruppe, welche

1 bis 6 Kohlenstoffatome umfasst, ist;
- $R^5$, das gleich oder verschieden ist, eine lineare oder verzweigte Alkylgruppe mit 1 bis 3 Kohlenstoffatomen oder eine Phenylgruppe ist; und
- $R^6$ für ein Wasserstoffatom, eine Gruppe $R^5$ oder ein freies Radlkal O° steht.

**6.** Zusammensetzung nach einem der vorangegangenen Ansprüche, **dadurch gekennzeichnet, dass** die Gruppe Y eine Gruppe der folgenden Formel (III) ist:

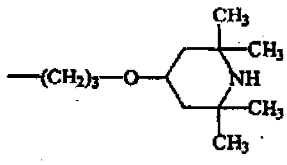

(III)

**7.** Zusammensetzung nach einem der vorangegangenen Ansprüche, **dadurch gekennzeichnet, dass** die Gruppe R, die gleich oder verschieden ist, eine Methyl-, Ethyl-, Isopropyl-, tert.-Butyl-, n-Hexyl-, Octyl-, Trifluorpropyl- oder Phenylgruppe ist.

**8.** Zusammensetzung nach einem der vorangegangenen Ansprüche, **dadurch gekennzeichnet, dass** R eine Methylgruppe ist.

**9.** Zusammensetzung nach einem der vorangegangenen Ansprüche, **dadurch gekennzeichnet, dass** das Polyorganosiloxan der Formel (I) ein lineares Polyorganosiloxan ist.

**10.** Zusammensetzung nach einem der vorangegangenen Ansprüche, **dadurch gekennzeichnet, dass** das Polyorganosiloxan der Formel (I) die folgende Formel (N) aufweist:

$$A\text{-}SiR_2\text{-}O\text{-}[SiRXO]_x\text{-}[SiRYO]_n\text{-}[SIR_2O]_m\text{-}O\text{-}SiR_2\text{-}A \qquad (IV),$$

in welcher:

- R, das gleich oder verschieden ist, eine derartige Gruppe, wie in einem der vorangegangenen Ansprüche definiert, ist,
- X, das gleich oder verschieden ist, eine derartige Gruppe, wie in einem der vorangegangenen Ansprüche definiert, ist,
- Y, das gleich oder verschieden ist, eine derartige Gruppe, wie in einem der vorangegangenen Ansprüche definiert, ist,
- A, das gleich oder verschieden ist, eine Gruppe R, Y oder X ist,
- x eine mittlere Zahl über 0 ist,
- n eine mittlere Zahl über 0 ist,
- m eine mittlere Zahl über 0 ist und
- x+m+n+2 = N.

**11.** Zusammensetzung nach Anspruch 10, **dadurch gekennzeichnet, dass**:

- A eine Gruppe R ist und
- R eine Methylgruppe ist.

**12.** Zusammensetzung nach einem der Ansprüche 10 oder 11, **dadurch gekennzeichnet, dass**:

- $m \geq 10$, vorzugsweise $m \geq 100$,
- $x \geq 1$ und
- $n \geq 3$.

**13.** Zusammensetzung nach einem der Ansprüche 10 bis 12, **dadurch gekennzeichnet, dass** das Polyorganosiloxan der Formel (I) zwischen 0,1 und 0,5 Gew.-% Stickstoffatome umfasst.

**14.** Zusammensetzung nach einem der Ansprüche 10 bis 13, **dadurch gekennzeichnet, dass**:

- $m \geq 50$
- $0{,}001 \leq n/(m+n+x) \leq 0{,}5$, vorzugsweise $0{,}005 \leq n/(m+n+x) \leq 0{,}1$.

**15.** Zusammensetzung nach einem der Ansprüche 10 bis 14, **dadurch gekennzeichnet, dass**:

- $m \geq 50$
- $0{,}001 \leq x/(m+n+x) \leq 0{,}5$, vorzugsweise $0{,}01 \leq x/(m+n+x) \leq 0{,}2$.

**16.** Zusammensetzung nach einem der vorangegangenen Ansprüche, **dadurch gekennzeichnet, dass** das Polyorganosiloxan der Formel (I) eine Viskosität zwischen 1000 und 1000000 mPa.s, vorzugsweise zwischen 5000 und 500000 mPa.s, vorzugsweise zwischen 10000 und 100000 mPa.s, aufweist.

**17.** Zusammensetzung nach einem der vorangegangenen Ansprüche, **dadurch gekennzeichnet, dass** das Polyorganosiloxan in der Zusammensetzung in Form einer Emulsion von das Polyorganosiloxan der Formel (I) umfassenden Tröpfchen, welche in einem kosmetisch verträglichen wässrigen Träger dispergiert sind, vorliegt.

**18.** Zusammensetzung nach einem der vorangegangenen Ansprüche, **dadurch gekennzeichnet, dass**:

- die Emulsion eine Emulsion ist, bei welcher die mittlere Größe der Tröpfchen über oder gleich 0,15 μm ist, welche mit Hilfe eines Emulgiermittels für das Polyorganosiloxan der Formel (I), vorzugsweise in einem Anteil unter 10 Gew.-% bezogen auf die Menge von Polyorganosiloxan der Formel (I), erhalten wird, oder
- die Emulsion eine Mikroemulsion ist, bei welcher die mittlere Größe der Tröpfchen unter 0,15 μm liegt, welche mit Hilfe eines Emulgiermittels für das Polyorganosiloxan der Formel (I), vorzugsweise in einem Anteil über 10 Gew.-% bezogen auf die Menge von Polyorganosiloxan der Formel (I), erhalten wird.

**19.** Zusammensetzung nach dem vorangegangenen Anspruch, **dadurch gekennzeichnet, dass** sie zusätzlich zu dem Polyorganosiloxan der Formel (I) umfasst:

- mindestens ein anionisches und/oder amphoteres grenzflächenaktives Mittel, allein oder in einer Mischung,
- gegebenenfalls mindestens ein Stabilisierungs- und/oder Konditionierungs- und/oder Konditionierungshilfsmittel oder eine Mischung von solchen Mitteln,
- gegebenenfalls ein anderes Polyorganosiloxan,
- gegebenenfalls einen UV-Filter.

**20.** Zusammensetzung nach Anspruch 19, **dadurch gekennzeichnet, dass** das Stabilisierungs- und/oder Konditionierungs- und/oder Konditionierungshilfsmittel ausgewählt ist unter:

- den kationischen Polymeren, die von Polysaccharlden abgeleitet sind, beispielsweise den kationischen Derivaten von Cellulosen, den kationischen Derivaten von Stärken, den kationischen Derivaten von Guar-Gummis, den kationischen Derivawen von Johannisbrotgumml,
- den synthetischen kationischen Polymeren,
- den Stabilisierungsmitteln, die vorzugsweise unter den vernetzten und festen unlöslichen Polyacrylaten, welche In der Zusammensetzung ein Netzwerk bilden, ausgewählt sind, beispielsweise Ethylenglycoldistearat (EGDS),
- den Mischungen oder Kombinationen von diesen Mitteln.

**21.** Zusammensetzung nach einem der vorangegangenen Ansprüche, **dadurch gekennzeichnet, dass** es sich um eine kosmetische Zusammensetzung für die Pflege der Haut und/oder der Haare, vorzugsweise für die Reinigung und/oder die Behandlung der Haut und/oder der Haare, handelt, wobei die Zusammensetzung In Form eines Fluids vorliegt.

**22.** Zusammensetzung nach einem der vorangegangenen Ansprüche, **dadurch gekennzeichnet, dass** es sich um ein Duschgel, ein Shampoo, ein nach dem Shampoo anzuwendendes Produkt, welches dazu bestimmt ist, ausgespült zu werden oder nicht, handelt.

**23.** Zusammensetzung nach einem der vorangegangenen Ansprüche, **dadurch gekennzeichnet, dass** es sich um ein Sonnenschutzprodukt handelt.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 6605577 B
- US 6642194 B
- WO 03088939 A
- WO 03066007 A
- US 5721297 A
- US 4565647 A
- WO 9858974 A
- WO 0075207 A
- WO 0142312 A
- WO 9801478 A
- WO 9903894 A
- WO 9931144 A
- WO 0226836 A
- WO 0210223 A
- WO 9630421 A


### Littérature non-brevet citée dans la description


- **M. L. Garcia ; J. Diaz.** Combability measurements on human hair. *J. Soc. Cosmet. Chem.,* 1976, vol. 27, 379
- **Y. K. Kamath ; H. -D. Weigmann.** Measurement of combing forces. *J. Soc. Cosmet. Chem.,* 1986, vol. 37, 111